(19) Europäisches Patentamt
European Patent Office
Office européen des brevets

(11) **EP 3 922 169 A1**

(12) **EUROPEAN PATENT APPLICATION**

(43) Date of publication:
**15.12.2021 Bulletin 2021/50**

(51) Int Cl.:
*A61B 5/00* (2006.01)          *A61B 18/02* (2006.01)
*A61B 34/10* (2016.01)         *A61B 34/20* (2016.01)
*A61B 90/00* (2016.01)         *A61B 5/06* (2006.01)

(21) Application number: **20179859.2**

(22) Date of filing: **12.06.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• PAPINI, Ilan
  5656 AE Eindhoven (NL)
• SCHWARTZ, Yitzhack
  5656 AE Eindhoven (NL)
• IBRAGIMOV, Zalman
  5656 AE Eindhoven (NL)

(74) Representative: **Philips Intellectual Property &
Standards
High Tech Campus 52
5656 AG Eindhoven (NL)**

(54) **GUIDING BALLOON THERAPY IN AN ANATOMICAL CAVITY**

(57)    A mechanism for generating two or more contact indicators indicating whether an inflatable balloon of a balloon therapy catheter is in contact with the boundaries of an anatomical cavity. The mechanism obtains an anatomical model of the anatomical cavity, determines a location of a balloon therapy catheter with respect to the anatomical cavity, and uses this information, together with one or more geometric properties of the inflatable balloon, to derive the two or more contact indicators.

FIG. 2

# EP 3 922 169 A1

**Description**

TECHNICAL FIELD

The present disclosure relates generally to guiding balloon therapy treatments, and, in particular, to systems and methods for guiding a balloon therapy treatment in a cavity of a subject.

BACKGROUND

Therapeutic balloons have been used recently to treat a variety of different ailments and disorders, including blood vessel occlusions and heart arrhythmias. For example, atrial fibrillation (AF) is an abnormal heart rhythm characterized by rapid and irregular beating of the atria. The disease is associated with an increased risk of heart failure, dementia, and stroke. AF may be caused by electrical pulses generated by secondary pacers at the ostium of a pulmonary vein (PV). Accordingly, one way of treating AF is by PV isolation, which can include ablating the inner wall of the left atrium (LA) to form lesions that isolate the ostium of the PVs from the rest of the LA.

Ablation can be performed in various ways, including radiofrequency (RF) ablation, ultrasonic ablation, and cryoablation. RF ablation is a conventional ablation procedure that involves powering an RF electrode to create contiguous, transmural lesions using heat energy. Balloon-based therapy procedures, such as balloon angioplasty and balloon ablation can provide several advantages, including a shorter procedure time and the ability to treat an entire circumferential area (e.g., a blood vessel, PV ostium) with a single deployment. For example, in cryoablation, an inflatable cryoballoon is inflated, brought in contact with the ostium and cooled to a temperature (e.g., below -65° C) that causes an electrically-isolating lesion or firewall in the ostium tissue. In conventional systems, the cryoballoon is maneuvered to, inflated, and subsequently positioned at the ablation site to fully occlude blood flow form the PV to the LA before ablation by the cryocooling is started. In this way, it can be assured that the circular lesion around the circumference of the PV formed from the cryoablation will electrically isolate the PV from the LA. As another example, a RF balloon comprising a plurality of electrodes can be maneuvered to and positioned at the ablation site. The RF balloon is again positioned such that it fully occludes blood flow before the lesion is formed via RF ablation using the electrodes.

Some challenges with balloon therapy of either kind include guiding the ablation balloon (e.g., cryoballoon) to the ablation site, and ensuring that the balloon is placed and oriented to maintain contact with a full circumference of the ostium. If the balloon is misaligned during ablation, the resulting lesion can include one or more gaps which result in reconduction and recurrence of the arrhythmia thereby requiring a re-do procedure when the AF symptoms return.

In conventional methods, angiographic and/or fluoroscopic procedures are used to guide the balloon to the ablation site. Once the inflated balloon is in place, an X-ray absorbing contrast agent or dye is introduced into the PV upstream of the balloon to detect leaks or gaps in the interface between the balloon and the ostium of the PV prior to ablating the tissue. When gaps exist, a portion of contrast agent will leak through into the LA and this leaked portion can be detected under fluoroscopy, which indicates that the balloon is not yet fully occluding blood flow from the PV into the LA, and therefore is not optimally positioned to completely isolate the PV from the LA. Accordingly, the physician can adjust the balloon until no residual leaks of the contrast agent are seen.

There are some shortcomings to using angiography and contrast agent to guide balloon ablation procedures. For example, patients and physicians may prefer to avoid x-ray radiation emitted during angiography and fluoroscopy procedures. Furthermore, guiding the balloon to the ablation site may be an imprecise and difficult processes that requires special expertise. It may be unadvisable to use contrast agent for some patients, and the contrast agent may not adequately identify leaks between the ostium of the PV and the balloon. For example, at least 20% of the population has some type of contraindication for contrast agents, including allergic reactions and kidney failure.

SUMMARY

The invention is defined by the claims. The independent claims provide aspects of the present disclosure in the form of devices, systems, methods, computer programs and media comprising the computer programs for assisting or guiding a balloon therapy procedure, such as a balloon ablation procedure. Such procedures are typically conducted using a balloon of a balloon therapy catheter for contacting a boundary of an anatomical cavity of a subject during the procedure. The aspects may be further defined with corresponding features. The features of one aspect may be used to define or further define the features of another and may provide corresponding advantages to the aspects involved.

The present disclosure proposes a mechanism for generating information on how an inflatable balloon (used to perform balloon therapy within an anatomical cavity of a patient, such as a heart chamber or blood vessel) interacts with the boundaries of the anatomical cavity. In particular, the disclosure proposes a mechanism for determining two or more indicators of whether the inflatable balloon makes contact with the boundaries of the anatomical cavity. This enables an assessment as to whether or not the inflatable balloon is occluding the anatomical cavity and to what extent.

Generating this information assists a clinician to more precisely align the balloon therapy catheter within the subject, to thereby improve the technical process of performing balloon therapy on a subject. Moreover, the information provides information on the internal state of the subject and/or the balloon therapy catheter with respect thereto.

In a first aspect there is provided a device for assisting a balloon therapy procedure conducted using a balloon of a balloon therapy catheter for contacting a boundary of an anatomical cavity of a subject during the procedure, the device comprising:

- a processor circuit comprising:

  - a first input configured to receive model data representative of an anatomical model of the anatomical cavity;
  - a second input configured to receive location data representative of a location of the balloon within the anatomical cavity during the procedure;
  - a third input configured to obtain geometrical data comprising one or more geometrical parameters associated with the balloon and representative of a balloon model of the balloon in inflated state;

- a data processor communicatively coupled to the first, second and third inputs and configured to:

  - determine, using the anatomical model, the balloon model, a relative position of the balloon model with respect to the anatomical model;
  - generate, using the anatomical model, the determined relative position and the balloon model, two or more contact indicators each one indicating a state of contact relating to one of two or more surface portions of the balloon when in an inflated state with the boundary of the anatomical cavity; and

- optionally, an output, communicatively coupled to the data processor, the output configured for outputting a signal carrying contact information comprising the two or more contact indicators.

The model data may be generated by an imaging system. The location of the balloon may be a location of the balloon in inflated and/or deflated state. The two or more surface portions at least partially do not mutually overlap. Preferably they do not overlap mutually.

In embodiments or examples, the two or more contact indicators comprises ten or more contact indicators, or even 20 or more contact indicators. A corresponding number of respective balloon portions exists in such cases.

In some embodiments or examples, the state of contact comprises an estimation of whether or not a respective portion of the inflatable balloon is in contact with a boundary of the anatomical cavity.

In embodiments or examples, the balloon is part of and affixed to a balloon therapy catheter which also comprising an EP catheter.

In some embodiments or examples, the processor circuit further comprises an output configured to provide the signal carrying contact information responsive to the two or more contact indicators.

In some embodiments the two or more contact indicators comprises one or more of: a distance indication of a distance between an edge of the respective portion of the balloon in the inflated state and the boundary of the anatomical cavity and a pressure indication of a pressure applied by the respective portion of the balloon in the inflated state to the boundary of the anatomical cavity.

In some embodiments and examples, each of the two or more contact indicators comprises a distance indication and a pressure indication.

In some embodiments the device of any one of the previous claims, wherein the processor circuit is configured to :

- generate, using the one or more geometrical parameters, the balloon model.

In some embodiments or examples, the generation of the balloon model form the balloon data is performed before the relative position of the balloon model with respect to the anatomical model is determined.

In some embodiments the device of any one of the previous claims, wherein the processor circuit is configured to:

- determine one or more reference points within the balloon envelope of the balloon model;
- assign or relate to each of the two or more balloon portions a particular surface portion of the balloon model that bounds the balloon portion;
- for each particular surface portion, define a virtual axis intersecting a determined reference point and a surface point of intersect lying within the particular surface portion and determine a surface portion distance as the distance between the determined reference point and the surface point of intersect;
- for each particular surface portion, determine a boundary point of intersect of the virtual axis of a particular surface portion with the cavity boundary of the anatomical model and determine a boundary distance as the distance between the determined reference point and the boundary point of intersect defining the virtual axis of that particular surface portion:

- generate, based on the surface portion distances and the corresponding boundary surface distances, the two or more contact indicators.

In some embodiments or examples the step of generating the two or more contact indicators comprises for each one of the indicators calculating a difference between surface portion distance and boundary distance for a particular surface portion.

In some embodiments of examples the two surface portions are each part of a balloon segment representing a cross-section of the balloon wherein the cross-sections are parallel to one another, optionally where the reference points, surface intersect point and boundary intersect points are defined such that the virtual axes of different segments are parallel to each other. In the optional case the reference points are different. The reference points preferably are defined to be part of a central axis parallel to and coinciding with the balloon therapy longitudinal axis within the balloon model.

In some examples or embodiments each balloon segment comprises at least two radial sections of a cross-section such that a surface portion is defined by a radial section within a segment. Preferably each cross-section extends normal to a central axis parallel to and coinciding with the balloon therapy catheters longitudinal axis within the balloon model.

In some examples or embodiments the processor circuit is further configured to:

- be able to communicate with one or more of a plurality of electrodes of an EP catheter and with a plurality of external body patch electrodes for positioning on a subject to apply an electrical field to at least the anatomical cavity using body patch electrical signals;
- be able to control the external body patch electrodes to apply the electrical field;
- be able to control the one or more of the plurality of electrodes to:

  - generate the location data;
  - and, optionally, generate the model data.

According to a further aspect there is provided a system for assisting a balloon therapy procedure conducted using a balloon of a balloon therapy catheter for contacting a boundary of an anatomical cavity of a subject during the procedure, the system comprising:

- a device of any of the previous claims wherein the processor circuit comprises an output communicatively coupled to the data processor; and
- a user interface configured to provide an indication of the contact information to the user.

The user interface may be communicatively coupled to the processor circuit at least via the output to this end.

The processor circuit can be configured to provide a visual representation of the contact information and the user interface can be configured to provide the visual representation to the user, for example by a display as part of the user interface.

In some embodiments or examples the system, comprises:

- the balloon therapy catheter; and
- an electrophysiology catheter comprising one or more of a plurality of electrodes.

In a further aspect there is provided a method for assisting a balloon therapy procedure conducted using a balloon of a balloon therapy catheter for contacting a boundary of an anatomical cavity of a subject during the procedure, the method comprising:

- receiving, at a first input of a processing circuit, model data representative of an anatomical model of the anatomical cavity ;
- receiving, at a second input of the processing circuit, location data representative of a location of the balloon within the anatomical cavity during the procedure ;
- receiving, at a third input of the processing circuit, geometrical data comprising one or more geometrical parameters associated with the balloon and representative of a balloon model of the balloon in inflated state;
- determining, by a data processor of the processor circuit and communicatively coupled to the first, second and third inputs, using the anatomical model and the balloon model, a relative position of the balloon model with respect to the anatomical model;

- generating, by the data processor, using the anatomical model, the determined relative position and the balloon model, two or more contact indicators each indicating a state of contact of a respective portion of the balloon when in an inflated state with the boundary of the anatomical cavity; and
- optionally, at an output of the processing circuit communicatively coupled to the data processor, a signal carrying contact information comprising the two or more contact indicators.

In some embodiments or examples of the method the two or more contact indicators comprises one or more of: a distance indication of a distance between an edge of the respective portion of the balloon in the inflated state and the boundary of the anatomical cavity and a pressure indication of a pressure applied by the respective portion of the balloon in the inflated state to the boundary of the anatomical cavity.

In some embodiments or examples the method comprises :

- generating, by the processing circuit, using the one or more geometrical parameters, the balloon model.

In some embodiments or examples the method the step of generating the two or more contact indicators comprises:

- determining one or more reference points within the balloon envelope of the balloon model;
- assigning or relateing to each of the two or more balloon portions a particular surface portion of the balloon model that bounds the balloon portion;
- for each particular surface portion, defining a virtual axis intersecting a determined reference point and a surface point of intersect lying within the particular surface portion and determining a surface portion distance as the distance between the determined reference point and the surface point of intersect;
- for each particular surface portion, determining a boundary point of intersect of the virtual axis of a particular surface portion with the cavity boundary of the anatomical model and determining a boundary distance as the distance between the determined reference point and the boundary point of intersect defining the virtual axis of that particular surface portion:
- generating, based on the surface portion distances and the corresponding boundary surface distances, the two or more contact indicators.

In some embodiments or examples of the step of generating the two or more contact indicators comprises for each one of the indicators calculating a difference between surface portion distance and boundary distance for a particular surface portion.

In some embodiments or examples the two surface portions are each part of a balloon segment representing a cross-section of the balloon wherein the cross-sections are parallel to one another, and, optionally, wherein the reference points, surface intersect point and boundary intersect points are defined such that the virtual axes of different segments do not intersect but only cross each other or are parallel.

The reference points preferably are defined to be part of a central axis parallel to and coinciding with the balloon therapy longitudinal axis within the balloon model.

In some embodiments or examples each balloon segment comprises at least two radial sections of a cross-section such that a surface portion is defined by a radial section within a segment.

Preferably each cross-section extends normal to a central axis parallel to and coinciding with the balloon therapy catheters longitudinal axis within the balloon model.

In some embodiments or examples of the method the processing circuit is configured to communicate with one or more of a plurality of electrodes of an EP catheter and with a plurality of external body patch electrodes for positioning on a subject to apply an electrical field to at least the anatomical cavity using body patch electrical signals, and the method comprises:

- controlling the external body patch electrodes to apply the electrical field;
- controlling the one or more of the plurality of electrodes to:

  - generate the location data;
  - and, optionally, generate the model data.

According to a further aspect there is provided a computer program product comprising code which, when executed by a processor circuit and/or data processor of the device of any of the devices or systems defined herein, causes the processor circuit and/or the data processor to perform the steps of any one of the methods as defined herein. The processor circuit may be part of a computer, workstation, or any system described herein.

According to a further aspect there is provided a computer-readable medium or data carrier comprising or carrying the computer program product. The computer readable medium may be a non-transitory medium. The non-transitory medium may be comprised within the processor circuit and/or a system as described herein. The non-transitory medium may comprise a memory as described herein.

Further definitions and advantages are provided herein below.

The determined position of the inflatable balloon may define how the inflatable balloon is arranged with respect to the anatomical cavity. By way of example only, the determined position of the inflatable balloon may include a determined (point) location of the inflatable balloon (e.g. a position of an end, center or central axis of the balloon) and/or an orientation of the inflatable balloon with respect to the anatomical model.

It will be apparent that the (point) location of the inflatable balloon can be directly derived from the location data.

Orientation information may indicate a relative orientation of the inflatable balloon (with respect to the anatomical model). Orientation information may be derived from the location data from the location data, e.g. by tracking a path of the balloon. For example, the processor circuit may use a path taken by the inflatable balloon as it moves with respect to the anatomical model to determine or predict an orientation of the inflatable balloon, i.e. generate orientation information. A radial component of a movement or path can be used to account for sideways movement (compared to a back and forth movement).

In other examples, orientation information may be obtained from a separate source, such as an accelerometer (or other orientation sensor) mounted on the balloon therapy catheter.

In some examples, the model data is obtained from an imaging system. Imaging systems may be capable of imaging a subject to generate an anatomical model of an anatomical cavity of the subject. Example imaging systems may use any suitable imaging technology, such as MRI, CT or ultrasound imaging techniques. Preferably, the imaging system is a dielectric imaging system that uses a dielectric imaging technique.

In some examples, the location data is obtained from a location system. A location system is configured to determine a relative location of the catheter and/or the inflatable balloon with respect to the anatomical model. Suitable location systems may employ a magnetic sensor (e.g. mounted on the catheter) and/or one or more electrodes mounted on the catheter to identify a relative location of the catheter and or inflatable balloon (e.g. using dielectric monitoring approaches).

In embodiments, the location data uses a same co-ordinate system as the model data. For example the imaging system and location system my operate such as to output model data and location data in the same co-ordinate system. In some embodiments the co-ordinate systems are different and a relationship between the co-ordinate systems is known and/or calculable. In such case the processor may be configured to transform one or more of the model data and location data to a same co-ordinate system, for example using the known or calculable relationship mentioned hereinbefore.

In some embodiments, the model data and location data may be obtained in the same parameter space. For example, both data may comprise physical locations in three-dimensional space. In another example the data may comprise the same physical parameters in three-dimensional space where each of the parameter spaces is representative of the physical locations in three dimensional space, but is not actually consisting of physical location data in three dimensional space. In other embodiments, the parameter spaces of the different data entirely different. In such cases where one or more parameter spaces are different from the physical location space, the processor may be configured to transform one or more of the non-location spaces into the same physical location space. For example, the model data may comprise electrical and/or dielectric data in a three-dimensional space, i.e. electric field in three-dimensional space. Such data may be obtained using a dielectric imaging system (as will be described herein). The processor may be configured to transform the electrical and/or dielectric data into the physical location space.

Of course, the model data and/or location data can be obtained from other sources, such as a memory, storage unit or other processing system/module. In such case they may have been recorded or determined using the appropriate systems at another time such as pre-operative.

In some examples, the imaging system and the location system are combined or integrated into a single system. In this way, the single system is capable of both generating an anatomical model of the anatomical cavity and tracking a relative location of the balloon within the anatomical cavity. In such case the processor circuit or processor may be a distributed circuit or processor each part serving the respective imaging system and location system. Alternatively, the processor circuit or processor may be a single circuit serving both the imaging system and the location system.

In some examples, the geometrical data is obtained from a memory, storage unit and/or a user interface. The geometrical data represents parameters of the inflatable balloon. In embodiments it comprises parameters of a partially and/or fully inflated balloon or for calculating such. It preferably comprises at least the parameters of a fully inflated balloon.

The processor circuit may further comprise an output configured to provide the signal carrying contact information responsive to the two or more contact indicators. Thus, the information generated by the processor circuit may be made available to an external module or system, such as a user interface or further processing system.

Preferably, the two or more contact indicators comprises ten or more contact indicators, or more preferably, twenty or more contact indicators or, more preferably, thirty of more contact indicators.

Preferably, each contact indicator represents a prediction of whether a respective portion of the inflatable balloon is in contact with a boundary of the anatomical cavity. In this way, the relationship between different parts of the inflatable balloon and the anatomical cavity can be determined. This makes additional information available to a user that could result in improved understanding of the relationship between the anatomical cavity and the inflatable balloon (e.g. an improved understanding of the state of the inflatable balloon and the subject). This additional information may be made available to a user/clinician or further processed to identify yet further clinically useful information about the relationship between the inflatable balloon and the anatomical cavity.

Each of the two or more contact indicators may be a measure responsive to a predicted pressure applied by the portion of the inflatable balloon to the boundary of the anatomical cavity and/or a predicted distance between an edge of the portion of the inflatable balloon and the boundary of the anatomical cavity.

The processor circuit may be configured to generate the two or more contact indicators by: generating, using the one or more geometrical parameters associated with the inflatable balloon, a model of the inflatable balloon; generating the two or more contact indicators by processing the model of the inflatable balloon, the anatomical model of the anatomical cavity and the determined location of the inflatable balloon with respect to the anatomical model.

In some examples, the two or more portions of the inflatable balloon comprise at least two segments each of a plurality of cross-sections of the inflatable balloon, and preferably at least ten such segments and more preferably at least twenty such segments. Having at least 10 segments provides improved modeling of a flexible balloon around its circumference. Preferably, in such examples, there are no fewer than two cross-sections, preferably no fewer than ten cross-sections and more preferably no fewer than twenty cross-sections. Embodiments having at least ten segments each having at least 10 cross-sections are preferred. They may provide an improved leak detection.

Optionally, each of the plurality of cross-sections of the inflatable balloon are parallel to one another.

Preferably, the at least two segments of each of a plurality of cross-sections comprises at least two radial segments of each of the plurality of cross-sections. Preferably, each segment of a cross-section is a radial segment of a cross-section. The balloon therapy catheter may have a central axis, and each cross-section of the balloon may be arranged so that the central axis is normal to each cross-section (i.e. parallel to one another).

In some examples, the position of the balloon therapy catheter is a position of a center or central axis of the balloon therapy catheter; and the processor circuit is configured to generate the two or more contact indicators by: determining, using the one or more geometrical properties of the inflatable balloon, a respective distance between the center or central axis and an edge of the inflatable balloon in each of two or more directions from the center or central axis of the inflatable balloon; determining, using the anatomical model of the anatomical cavity and the determined location of the center or central axis of the inflatable balloon with respect to the anatomical cavity, a distance between the center or central axis and the boundary of the anatomical cavity in each of the two or more directions from the center or central axis of the inflatable balloon; and generating, for each of the two or more directions from the center or central axis of the inflatable balloon, a contact indicator that predicts whether the inflatable balloon is in contact with the boundary of the anatomical cavity in the said direction by processing the distance between the center or central axis and an edge of the inflatable balloon in said direction and the distance between the center or central axis an the boundary of the anatomical cavity in said direction.

In some examples, the step of generating, for each of the two or more directions from the center or central axis of the inflatable balloon, a contact indicator comprises, for each of the two or more directions from the center or central axis of the inflatable balloon: subtracting the distance between the center or central axis and an edge of the inflatable balloon in said direction from the distance between the center or central axis an the boundary of the anatomical cavity in said direction, to thereby generate a measure responsive to a predicted pressure applied by the portion of the inflatable balloon to the boundary of the anatomical cavity and/or a gap between the inflatable balloon and the boundary of the anatomical cavity.

Thus, the measure provides a numerical indicator of the amount of pressure applied by a portion of the inflatable balloon to the boundaries of the anatomical cavity and/or a gap between the portion of the inflatable balloon and the boundaries of the anatomical cavity.

In some examples, the processor circuit or processor is further configured to: process the two or more contact indicators to predict a center of pressure applied by the inflatable balloon to the boundary of the anatomical cavity; and output a signal carrying contact information responsive to the predicted center of pressure.

The processor circuit or processor may be configured to output the signal carrying the contact information to a user interface communicatively connected with the processor circuit.

There is also proposed a system for assisting a balloon ablation procedure in which a balloon therapy catheter comprising an inflatable balloon is used where the assisting comprises generating contact information indicating whether the inflatable balloon is in contact with a boundary of an anatomical cavity of a subject during the procedure, the system comprising: the processor circuit previously described; and, optionally, a user interface configured to communicatively connect to the processor circuit and to provide one or more of an audible or visual representation of the contact information to a user. This system is capable of providing the contact information to a user to thereby assist a user during an ablation procedure. A user may be one or more of a system operator, caregiver, physician or the subject.

There is also proposed a method of guiding a balloon assisted ablation procedure using a balloon therapy catheter comprising an inflatable balloon by generating contact information indicating whether the inflatable balloon is in contact with a boundary of an anatomical cavity of a subject during the procedure.

The method comprises: obtaining model data comprising an anatomical model of the anatomical cavity generated by an imaging system; obtaining location data responsive to a change in position of the inflatable balloon within the anatomical cavity during the ablation procedure; determining, using the anatomical model and the location data, a position of the inflatable balloon with respect to the anatomical model; obtaining geometrical data comprising one or more geometrical parameters associated with the inflatable balloon; generating two or more contact indicators using the anatomical model, the determined position of the inflatable balloon and the one or more geometrical parameters; and outputting a signal carrying contact information responsive to the two or more contact indicators.

Preferably, the method is computer-implemented. The method may be carried out by a processor circuit or a processor thereof, such as the processor circuit and processor previously described. In some embodiments the processor circuit is capable of obtaining the different data and processing the obtained data. In some embodiments the processor circuit is further capable of controlling a plurality of electrodes of an EP catheter (e.g. of a balloon therapy catheter) and a signal processor to which the EP catheter can be or is connected where the plurality of electrodes and the controller are controlled to generate the location data and optionally also the model data. While the location data are typically generated during the balloon ablation procedure, the model data may be generated pre-operatively, although they may be advantageously generated during the procedure for example before the generation of the location data.

Each contact indicator may represent a prediction of whether a respective portion of the inflatable balloon is in contact with a boundary of the anatomical cavity. Preferably, each contact indicator is a measure responsive to a predicted pressure applied by the portion of the inflatable balloon to the boundary of the anatomical cavity and/or a predicted distance between an edge of the portion of the inflatable balloon and the boundary of the anatomical cavity.

The method may comprise a step of providing an audible or visual representation of the contact information. The audible or visual representation may be provided using a user interface connected to the processor circuit with which the method is performed.

The skilled person would be readily capable of adapting any herein described method to reflect embodiments of herein described apparatus, systems and/or processors, and vice versa. A similar understanding would be made by the skilled person with respect to a computer program (product).

Additional aspects, features, and advantages of the present disclosure will become apparent from the following detailed description.

BRIEF DESCRIPTION OF THE DRAWINGS

Illustrative embodiments of the present disclosure will be described with reference to the accompanying schematic drawings, of which:

Fig. 1 is a depiction of catheter assembly positioned at the PV ostium with complete occlusion;
Fig. 2 illustrates a dielectric imaging system for use in embodiments of the invention;
Fig. 3 illustrates a balloon therapy system;
Fig. 4 illustrates a balloon therapy system;
Fig. 5 provides a flowchart according to an embodiment;
Fig. 6 illustrates a balloon therapy catheter according to an embodiment;
Fig. 7 demonstrates radial segments of a section of an inflatable balloon forming portions of the inflatable balloon;
Fig. 8 illustrates an alternative method of conceptually dividing a balloon of a therapy catheter;
Fig. 9 is a visual representation of information obtained from contact indicators generated by embodiments;
Fig. 10 is a visual representation of a display for an embodiment; and
Fig. 11 illustrates a processor circuit.

DETAILED DESCRIPTION

For the purposes of promoting an understanding of the principles of the present disclosure, reference will now be made to the embodiments or examples illustrated in the drawings, and specific language will be used to describe the same. It is nevertheless understood that no limitation to the scope of the disclosure is intended. Any alterations and further modifications to the described devices, systems, methods, computer programs and any further application of the principles of the present disclosure are fully contemplated and included within the present disclosure as would normally occur to one skilled in the art to which the disclosure relates.

The present invention proposes a mechanism for generating two or more contact indicators for two or more respective portions of the balloon each indicating a state of contact between the inflated balloon and the boundaries of an anatomical cavity. Such indication may for example be whether or not there is contact and/or if there is contact how firm such contact is for example by providing a pressure or force indication.

The mechanism is based on the use or generation of an anatomical model comprising the anatomical cavity, a location of at least one point of a balloon therapy catheter having fixed thereto a balloon with respect to the anatomical cavity, together with one or more geometric properties of the balloon, to derive the two or more contact indicators. This is advantageous as a local (segmental) state of contact between parts or portions of the balloon (surface) and the wall may be estimated so as to assist a user in a therapy procedure. For example, in a balloon ablation procedure it may be important to have a defined minimum contact in order to simplify execution of the procedure and/or improve the coarse of the procedure and/or possibly improve its outcome. As will be described, the mechanism allows such estimations to be done without the use of X-ray during the procedure and with the use of a dielectric imaging system with which a location of the balloon can be determined through location determination of one or more electrodes of an electrophysiology catheter. Since such EP catheters are currently typically used in combination with a balloon therapy catheter to perform the procedure, no changes to existing catheters need be made. One difficulty with many of the balloon therapy catheters is that the balloon is, due to its material constitution difficult to localize using e.g. the dielectric imaging. Hence direct visualization of a state of contact is difficult. The mechanism at least partly helps to reduce that problem.

As mentioned above, fluoroscopy-based approaches used in guiding balloon therapy procedures, including navigation and deployment of the balloon at the therapy site (e.g., blood vessel stenosis, PV ostium), suffer from various drawbacks. It may be desirable to provide an approach for generating information that can help a clinician to perform a balloon treatment procedure without using fluoroscopy and/or contrast agent. The present disclosure provides systems, methods, and devices for generating information to aid in a balloon therapy procedure using the aforementioned mechanism. As will be further described below, a system can be used to provide information that aids in the placement of an inflatable balloon, such as an ablation balloon, an angioplasty balloon, or a scoring balloon with respect an anatomical cavity, such as the LA or a blood vessel.

The devices, systems, and methods described herein are applicable to a variety of treatment procedures in which a balloon is used to occlude a body lumen or body cavity. Although the following disclosure may refer to embodiments that include balloon therapy procedures, cryoablation, cyroballoons, cryocatheters, balloon angioplasty, RF balloon ablation, or RF balloons, it will be understood that such embodiments are exemplary, and are not intended to limit the scope of the disclosure. The anatomical cavity may for example be a vessel and in particular is one that is connected to a heart chamber such as the atria. The vessel may thus be a pulmonary vein (PV). The boundary may be a vessel wall and this may be near the ostium of for example a PV.

It is contemplated that the features, components, and/or steps described with respect to one embodiment may be combined with the features, components, and/or steps described with respect to other embodiments of the present disclosure. For the sake of brevity, however, the numerous iterations of these combinations will not be described separately.

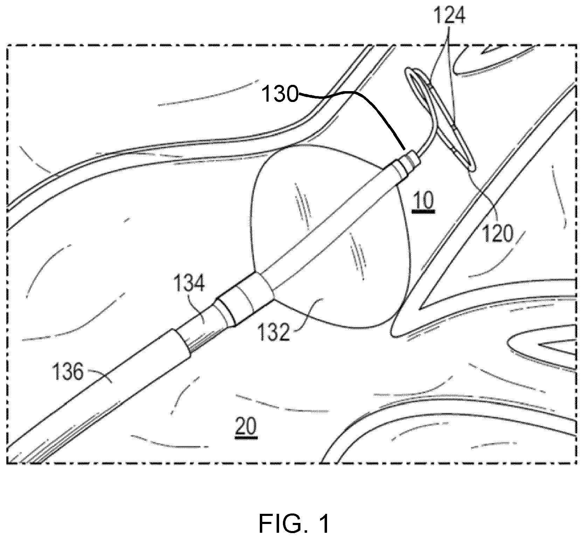

Fig. 1 provides an exemplary embodiment of a catheter assembly that may be used in an ablation therapy. The assembly includes a balloon therapy catheter 140 having an inflatable balloon 132 shown in inflated condition and positioned at the ostium of an anatomical cavity exemplified with that PV 10 within an overall anatomical cavity 20 representing the left atrium (LA) and PVs connected thereto of heart of a subject. The balloon therapy catheter in this case is a balloon ablation catheter. The skilled person would appreciate other use case scenarios and positions for balloon therapy.

The balloon therapy catheter comprises an inflatable balloon (shown in inflated condition) 132 attached to a distal portion of the flexible elongate member 134. The elongate member 134 has a member tip 130 at a distal position to the balloon 132.

In this example the balloon 132 is a cryoballoon configured to be inflated and then at least partially filled with a cooling fluid (refrigerant) to cool the cryoballoon to a temperature (e.g., below -65° C) that causes an electrically-isolating lesion or firewall in the tissue when in contact with the boundary of the cavity. For example, the cryoballoon 132 may be in fluid communication with a source or reservoir of cooling fluid via one or more fluid lines positioned within a flexible elongate member 134. Further, the cryoballoon 132 may be in fluid communication with an air or gas source, e.g., for balloon inflation, via one or more fluid lines positioned within the flexible elongate member 134.

The elongate flexible member 134 comprises a lumen extending from its proximal end (not shown) to its distal tip 130 and is suitable for slidably receiving an EP catheter 120, sometimes also referred to as a mapping catheter. The EP catheter comprises one or more (e.g. a plurality of) separately addressable electrodes 124 disposed along a distal tip thereof. The EP catheter is extendible from or retractable into the lumen via the tip 130 of the flexible member 134. The EP catheter may be removed from the member 134 entirely if needed.

The elongate member is movably disposed within the sheath 136. When the balloon is deflated the whole of the member 134 including the deflated balloon may be retracted into and through the sheath 136. When protruding out a distal end of a flexible steering sheath 136 , the balloon may be inflated as shown. The flexible steering sheath 136 has pull wires to be operated by a user to steer the sheath and guide the balloon therapy catheter as well as the EP catheter if contained therein.

The EP catheter 120 comprises one or more (a plurality of) mutually electrically separated electrodes 124 disposed along an elongate catheter tip member 126. Only three of those are shown in the Fig. 1 for clarity only. In some embodiments, the EP catheter 120 comprises between 8 and 10 electrodes. However, the catheter can include other numbers of electrodes, including 2, 4, 6, 14, 15, 20, 30, 60, or any other suitable number of electrodes. More than 10 electrodes are preferred and more than 15 are even more preferred.

Examples of Balloon therapy catheters manufactured by Medtronic™ are the Arctic Front™ Family of Cardiac Cryoablation Catheters and/or the FlexCath™ Advance Steerable Sheath. Examples of EP catheters that work with the aforementioned examples manufactured by the Medtronic™ are the **Achieve™ and Achieve Advance™ Mapping Catheters Families. However, other types of such cathters for exmaple from other manufacturers may be used in conjuction with the curretn mechanism.**

In some other embodiments, the one or more electrodes or even a second set of electrodes are mounted on the inflatable balloon. Such electrodes are typically used for providing ablation energy in the form of RF power. Further details regarding suitable catheters and assemblies can be found in, for example, U.S. Patent No. 6,002,955, titled "Stabilized Electrophysiology Catheter and Method for Use," the entirety of which is hereby incorporated by reference.

The movement of different catheters and sheaths may be controlled by a user using a catheter control device or handle. The handle is not shown in Fig. 1 and such control is known in the art. For example, the EP catheter may be controllably manually or via an automated mechanism to be retractable within and extendable from the lumen of the member 134 of the balloon therapy catheter 140. It may even be entirely removed from the lumen and for example replaced with a guide wire or other wire or catheter. The EP catheter 120 and therewith its electrodes may thus be moved relative to the inflatable balloon or member tip 130 for example. Likewise, the member 134 may be controlled by the handle and moved with respect to the sheath 136.

The EP catheter elongate tip member may be configured to be positioned distally of the cryoballoon for example during a therapy procedure, and may be biased, shaped, or otherwise structurally configured to assume a shape, such as a circular one in which the electrodes are spaced within one plane or a spiral one in which the electrodes are spaced from one another about one or more planes. For example, the elongate tip member can be similar to a spiral mapping catheter (SMC) in which electrodes are distributed along an elongate tip member having assumed or configurable to adopt a spiral configuration.

Using the controls described herein above, in some embodiments, a following procedure can be used. First the sheath 136 having retracted therein the balloon therapy catheter with balloon in deflated state and the EP catheter 120 is first introduced into the LA via known procedures to guide the balloon and EP catheter 120 to the ablation site. Alternatively, a guidewire taking the place of the EP catheter may first be introduced in the PV over which the balloon therapy catheter including the flexible member 134, deflated balloon and sheath 136 are guided towards the LA before the guide wire is replaced with the EP catheter. Once the balloon therapy catheter is in the LA, the flexible member 134 may be extended from the sheath 136 to expose the inflatable balloon 132 in order to inflate it as shown in Fig. 1 The EP catheter 120 may now be extended from the member 134 out of the tip 130 to be spiral or lasso shaped and positioned in the PV. Once positioned, the member 134 may be manipulated to bring the inflated balloon towards the PV ostium so that a full circumference of the balloon 132 is in contact with an entire circumference of the PV 10 near or at the ostium. In this way a full occlusion of the PV at the ostium may be achieved and by cooling of the balloon the ablation can be delivered around the entire circumference with an increased likelihood that the ablation results in complete electrical isolation.

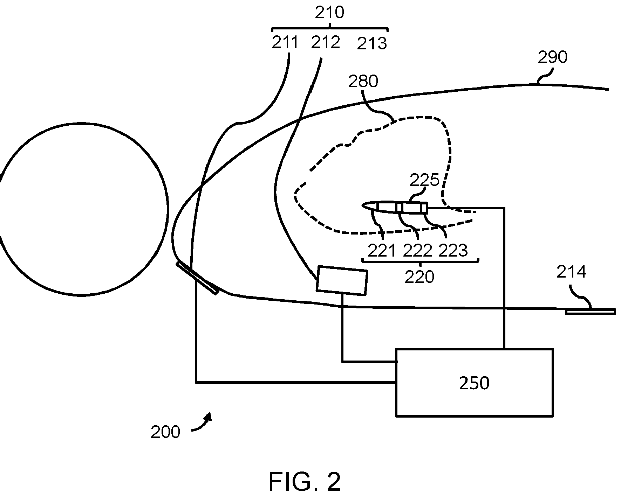

In some embodiments the one or more electrodes of the EP catheter may be used to map the anatomical cavity or parts thereof using a dielectric imaging or mapping system. The electrodes are then used to generate model data representative of an anatomical model of at least the anatomical cavity and its wall. This will be described in more detail with reference to Figs. 2 to 4.

In some embodiments one or more of the electrodes of the EP catheter may be used for determining and tracking a location and/or orientation of the catheter distal portion within the anatomical cavity, before, during and/or after the procedure. From that and for example a known relative position corresponding to the balloon 132 and the member tip 130 (which may be predefined or obtained using a position calibration procedure that will be described herein), a position of the balloon 132 within the cavity may be determined and tracked as will be described herein below.

In other embodiments of the present disclosure, the balloon therapy catheter comprises a different location/position sensor, such as a magnetic sensor, electromagnetic sensor or an integrated optical shape sensed fiber, which facilitates location and/or orientation determination and tracking thereof of the catheter and/or the inflatable balloon.

In these embodiments, any electrodes can be omitted from the balloon therapy catheter. Methods of tracking the location of a balloon within an anatomical cavity would be readily apparent to the skilled person and could be employed in embodiments of the present invention.

An example of a suitable magnetic tracking system is disclosed in European Patent No. 1,913,338, titled "System And Method For Magnetic Tracking Of A Sensor For Interventional Device Localization". An example of a suitable integrated optical shape sensed fiber is disclosed in U.S. Patent Application No. 2017/16462388 A, titled "Systems And Methods For Determining The Position Of A Non-Shape-Sensed Guidewire With A Shape-Sensed Catheter And For Visualizing The Guidewire". An example of a suitable electromagnetic sensor is disclosed in U.S. Patent Application No. 2016/15764094 A titled "Electromagnetic Navigation Device For Guiding And Tracking An Interventional Tool".

The approaches described in the present disclosure advantageously allow (visualized) assistance or guidance for the placement of a therapy (e.g. ablation) balloon and/or state of occlusion at the treatment site. Proposed approaches may facilitate improved occlusion of the PV at the ostium, by enabling contact between the balloon and the walls of the PV 10 to be checked.

For the purposes of this description, a location determination/orientation and tracking approach based on use of a dielectric imaging system is explained. The dielectric imaging system is capable of determining and tracking the position and/or orientation of the distal portion of the EP catheter using the location of the one or more electrodes dispose thereon. Embodiments using this type of tracking can make advantageous use of the mapping function of the system too and provide an integrated solution to the assistance or guidance of the procedure referred to herein above. The use of the same dielectric imaging system for both functions may omit the need for specially designed catheters (for example including specialized sensors or the like) and additional imaging or mapping systems. However, such integration is not necessary per se for benefitting from the use of the mechanism disclosed and the imaging or mapping and/or the tracking may be done differently in other embodiments as will be described herein below.

The operation of an example dielectric imaging system is now described first with reference to Fig. 2 to improve a contextual understanding of embodiments.

Fig. 2 illustrates a dielectric imaging system 200 (which may also be referred to as a dielectric mapping system) for generating an anatomical model of an anatomical cavity 280, (such as a chamber, blood vessel and/or heart chamber or other void) using a dielectric imaging process. The system may determine such model within a subject 290 by measuring the influence of tissue surrounding the anatomical cavity on a global electric field applied to the cavity via external electrodes 210 (body/patch electrodes) applied to the external of the subject and a local electric field applied and measured by the electrodes of the EP catheter 225 located within the cavity of the subject. The combined generation of fields and measurement of distortions of the electric fields allows both the location of the internal electrodes to be determined and the boundaries, border or perimeter of an anatomical cavity to be mapped. The map may contain or represent an anatomical model of the anatomical cavity as will be explained in some more detail below.

In some more detail, the dielectric imaging system 200 comprises a first set 210 of three pairs of electrodes, 211, 212, 213 ("external electrodes") not all of which are shown in the Fig. 2 for clarity reasons. The pairs of external electrodes are to be positioned externally with respect to the subject 290 (e.g. patch electrodes provided on a skin of the subject). The pairs of electrodes may be mutually geometrically positioned on the subject such that imaginary axes each running between the electrodes of a respective pair are subsequently orthogonal with respect to one another (e.g. positioned substantially orthogonally to one another), so that any electric fields generated between each pair of electrodes are substantially orthogonal in for example an X, Y and Z coordinate system with respect to one another. There may be more than the six external reference electrodes. They don't need to be arranged in pairs orthogonally oriented.

There may be an external reference electrode 214 for example located relatively far away from the set of electrodes for setting a reference voltage. There may be more than one reference electrode if needed.

The external electrodes are positioned and operated to generate a global intra-body electrical field within the subject in a region comprising the cavity 280. The pairs of external electrodes may be operated at different frequencies in for example the frequency range of 20-100 kHz to be able to separate their respective contributions to the local fields measured by the catheter 225.

The dielectric imaging system 200 also comprises a second set 220 of electrodes 221, 222, 223 ("internal electrodes") positioned on a catheter 225 to be positioned within the anatomical cavity so that the electrodes are located within the anatomical cavity. The catheter may be any catheter having one or more (preferably a plurality) electrodes and may be an EP catheter stand alone, or as part of the catheter assembly as for example described herein above with reference to Fig 1.

A dielectric imaging control system 250 is configured to provide electrical signals to the external electrodes 210, and optionally receive signals therefrom. The control system is further configured to transmit electrical signals to the internal (catheter) electrodes 220 and to receive signals from these electrodes. The control system may thereto include a signal processor with hardware and software to generate and process the electrical signals under control of a processor circuit. For example it can comprise AD converters to generate data representative of transmitted and measured electrical signals such that these data can be provided to a processor circuit as incorporated for example in a computer or workstation that is part of the control system. The processor circuit is then capable of processing the data and derive the anatomical model and generate images thereof to be displayed to a user via a user interface such as for example a display communicatively coupled to the processor circuit. The controller may have a user interface coupled to the processor circuit for a user to provide control commands based on which the systems electrodes are operated.

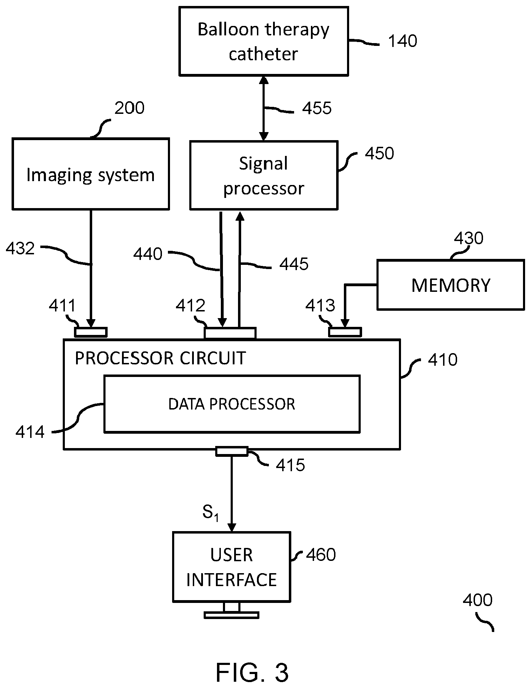

With the external global electrical field applied, the catheter 225 may then be used by a user to explore the cavity within the subject and the electrodes used to sense distortions in the global electrical field from which the control system generates model data representative of the anatomical model of the anatomical cavity. The model data so obtained typically contain electrical data such as electrical field or voltage map The controller is further configured to transform the electrical model data into a three-dimensional location/position coordinate cloud, such as for example a point cloud in Euclidian coordinates, which cloud is representative of the anatomical model. Optionally, the point cloud may be used to generate a mesh representing the 3D surface of the anatomical model using known methods in the art. For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 36. No. 1. 2017. The process of reconstructing an anatomical model from a point cloud is conceptually illustrated in Fig. 3, which demonstrates a process 350 in which a cloud of R-space points 310 ("point cloud") is transformed into an anatomical model 320. In the illustrated example, this is performed by creating a (3D) surface from the point cloud data, methods of which will be readily apparent to the skilled person. The entire process may be referred to as anatomical cavity mapping.

The control system 250 is optionally also configured to generate a visualization of the location of the electrodes 220 and therewith at least distal portion of the EP catheter 225. This may be done periodically to track a location of the catheter over time.

The dielectric imaging controller 250 thus can be used to generate an anatomical model of the anatomical cavity and can track a relative location of the internal electrodes 220 with respect to the anatomical cavity and anatomical model.

The control system typically is configured to output a visualization of the anatomical model of the anatomical cavity optionally together (e.g. as overlay) with the location of the catheter or at least the distal portion thereof comprising the electrodes 220. The anatomical model may be output to a display or other user interface (not shown).

The combined global and local field measurements enable detection of physiological movement associated with e.g. respiratory and cardiac motion and these can be corrected for using gating techniques when determining the anatomical model.

More detailed explanations of the use of body patch electrodes and catheter electrodes to generate the model data and locate the catheter or the distal portions thereof, gating and visualize the anatomical model and the locations of catheters are described in for example US patent 10,278,616 and US patent 5,983,126, or international patent application WO2018130974 and WO2019034944 the entireties of which are incorporated by reference.

The above-provided description of a dielectric imaging systems is only an example, and the skilled person would be readily capable of modifying the described dielectric imaging system appropriately using already known techniques in the field.

Other approaches for generating anatomical models will also be apparent to the skilled person, e.g. constructing an anatomical model of an anatomical cavity from ultrasound, MRI or CT data, which are known in the art and will not be described herein for brevity. Once an anatomical model of an anatomical cavity has been obtained in any way, e.g. via dielectric imaging, CT imaging, MR imaging US imaging or the like, then the location of internal electrodes with respect to the anatomical model can be readily defined since the electrode location may be tracked using any suitable tracking procedure.

Other methods for tracking the location of a catheter (and/or balloon) within an anatomical cavity will be apparent to the skilled person, such as those previously described.

Preferred embodiments of the present disclosure may however use the tracking capability of the dielectric imaging system, e.g. as described with reference to Figs. 2 to 4, to identify the relative location of the electrode(s) 124 of the EP catheter as then no specially designed catheters need be used. In this way, the relative position of the balloon therapy catheter and the balloon with respect to the anatomical model can be readily identified. Furthermore, if for the mapping also the dielectric imaging system is used, the position of the balloon therapy catheter is advantageously tracked in the same co-ordinate space as used to generate the anatomical model. This simplifies data processing and may make it even more accurate or less error prone.

In particular, a response of an electrode to an electric field may be used to identify a relative location of that electrode with respect to the anatomical map. This can be performed by transforming a response of an electrode to an electric field to a location co-ordinate.

Other approaches for using external electrodes (e.g. patch electrodes) and internal electrodes (e.g. catheter electrodes) to map body volumes and visualize the locations of catheters within the map can be found in the references cited herein before in relation to the mapping of the anatomical cavity.

Fig. 3 illustrates embodiments of a system for assisting or guiding a balloon assisted interventional procedure, such as for example balloon assisted ablation procedure. The system comprises a processor circuit 410. The processor circuit comprises a communications interface including a first input 411, an input/output (I/O) interface 412 serving as a second input and a third input 413. The system includes a signal processor 450 communicatively coupled to the processor circuit 410 via I/O interface 412 so that the processor circuit can obtain location data 440 from the signal processor and provide command data 445 to the Signal processor 450. The signal processor is also removably connected to the catheter 420 to exchange electrical signals. The balloon therapy catheter 140 may, for example, be embodied as previously described with reference to Fig. 1 and may comprises an inflatable balloon 132 and an EP catheter 120 having the one or more electrodes 124. The signal processor is connected to the electrodes 124 of the catheter to provide signals to the electrodes and receive electrical signals from the electrodes of the catheter. The signal processor contains appropriate hardware and software for processing the received electrical signals into location data, i.e. data representative of the location of the electrodes of the catheter 120 within a cavity. The signal processor may have analog to digital converters and the like to provide the location data in digital format that may be further processed by the processor circuit.

The system may also comprise the system for control of balloon assisted interventional procedures such as a RF- or cryoablation procedure. These systems are the ones to control the actual ablation and they are not shown in the Fig. although they could be partly or entirely integrated in the system. Such systems are known in the art and can be used without further adaptations.

The illustrated system of Fig. 3 is shown to work alongside or integrated with a an imaging system 200 that generates model data 432 representative of an anatomical model of an anatomical cavity. Thus, embodiments of the system and processing circuit do not need the presence of the imaging system, they just need to receive the model data 432. Such data may be obtained from e.g. ultrasound/MRI/CT imaging systems or other known modalities, but preferably are obtained from a dielectric imaging system. The model data may be provided to and obtained by the processor circuit.

In the illustrated example, information obtained from the dielectric imaging system 200 can be used together with the location data to track the position of an electrode on the catheter with respect to the anatomical model (using approaches previously described).

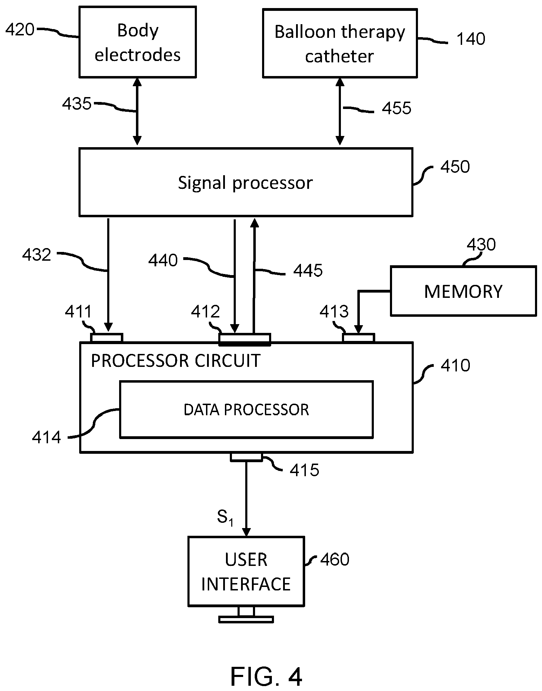

Some embodiments of the system may be described with reference to Fig. 4. In this case the imaging system is a dielectric imaging system integrated with the system.

The signal processor 450, processor circuit 410, memory 430 and optionally user interface 460 may for example be part of and partly or entirely the same as those within the control system 250 of the embodiment of Fig. 2.

In these embodiments, the signal processor 450 is also configured to communicate with the body electrodes to exchange electrical signals 435 and is configured to provide model data 432 to input 411 of the processor circuit 410. The model data are generated by the signal processor under control of the processor circuit by operation of the body patch electrodes 220 of the body electrodes 420 and the electrodes 124 of the EP catheter 120 of balloon therapy catheter 140 as described with reference of Figs. 1 and 2. This system conveniently integrates the mapping and location function without having to use specially designed catheters.

In the illustrated example, the model data is obtained from the (dielectric) imaging system 200, but could otherwise be obtained from a different system such as a memory or pre-processing module or network where such data have been stored when they have been pre-operatively recorded.

In embodiments such as those described with reference to Figs 3 and 4, geometrical data is obtained via input 413 for example from a memory 430. It may instead be obtained from a different module/unit, such as a user interface or have been stored in the memory after upload via the user interface. The geometrical data comprise data that describe geometrical aspects of the balloon and optionally its position with respect to the tip 130 of the balloon therapy catheter (Fig. 1) or provide data from which the processor circuit may calculate such geometrical aspects. Geometrical aspects may include for example size and shape in inflated condition or parameters from which such size and shape may be calculated. This will be described in more detail hereinafter.

The system may be a balloon therapy system 400 especially when it also includes the balloon therapy catheter 140.

In the embodiments shown and the system in general there may be electrode interfaces between body electrodes and EP catheter electrodes on one hand and the control system (via e.g. the Signal processor), but these are not shown. Communications and connections may be in wired or wireless form according to known methods. For example all connections to electrodes and between signal processor and processor circuit may be in the form of electrical wires or cables.

Thus, in embodiments of the system the processor circuit is configured to obtain model data comprising an anatomical model of the anatomical cavity at a first input 411, to obtain location data representative of a location of the catheter electrodes within the anatomical cavity (during the ablation procedure) at a second input 412 and to obtain geometrical data at a third input 413. It is noted that while the inputs have been separately shown and described, one or more and even all of them may be combined in one and/or may be part of the same I/O interface or communications module.

In embodiments, the processor circuit is part of a device which in one example is a computer of some kind such as a workstation, mobile computer of any type (phone, table laptop etc) such computer having all that is known in the art to make communications connections. The user interface 460 typically has at least one output device such as a display and at least one input device such as keyboard, mouse, joystick or foot peddle and may comprise any combination of these, to allow input of commands and data by a user.

The three pieces of data are processed, by the processing circuit and at least partly by the data processor 414, to generate the contact information comprising two or more contact indicators. Each contact indicator may represent an estimation of whether a portion of the inflatable balloon is in contact with a boundary (e.g. tissue) of the anatomical cavity. Contact indicators may thus comprise whether or not there is contact. Alternatively, or additionally a degree of contact may be provided in the form of e.g force, pressure or distortion parameter.

The processor circuit 410 may then output a signal S1 carrying contact information responsive to the generated two or more contact indicators. This signal may, for example, be provided at an output 415 of the processor circuit 410 and be passed to a user interface 460 communicatively coupled to the processor circuit so that for example a display of the user interface can display the contact information for assisting a user of the system during the procedure.

The current disclosure provides a process for assisting or guiding a balloon therapy procedure by generating contact information comprising at least two contact indicators. With this information an extent of contact between the inflatable balloon and a boundary of an anatomical cavity of a subject may be provided to a user to assist him in performing the balloon based therapy.

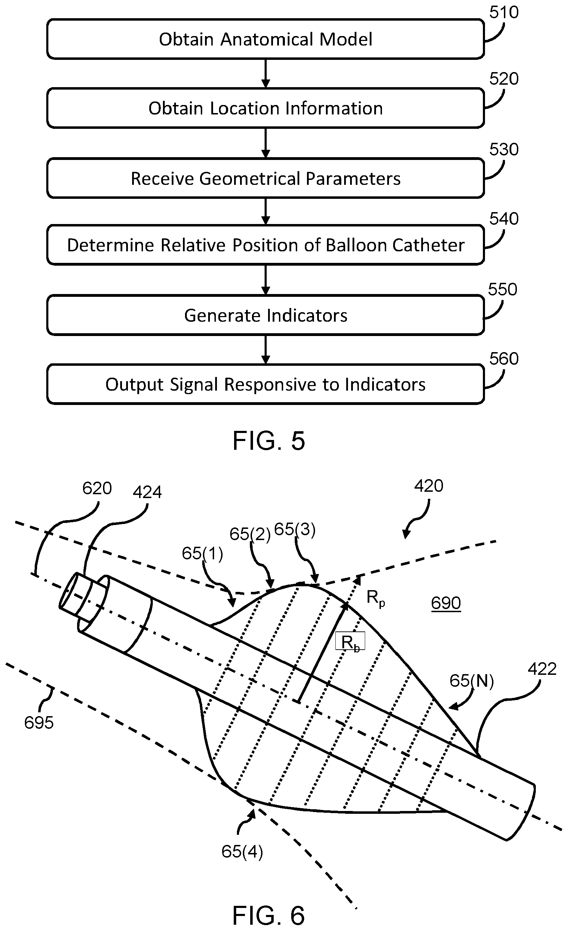

Fig. 5 illustrates such process 500. Embodiments of the systems as described herein for example with respect to Figs 1 to 4 can be used to perform this process.

In particular the process may be entirely or partially performed by the processor circuit 410 (and at least partly the data processor) and any system having such circuit in order to generate the contact information. Thus, although the process relates to an interventional procedure and may be performed during an interventional procedure it is itself not considered an interventional procedure. The process may be a computer implemented process or method.

In some examples, the process 500 or a part or one or more steps thereof are only performed after the inflatable balloon has been inflated. Such inflation may be done by a separate control system which was indicated not to be shown in Figs. 3 and 4. These are known in the art and are manufactured by for example Medtronic™.

The process 500 comprises a step 510 of obtaining (e.g. at the first input) model data representative of, comprising, or containing an anatomical model of the anatomical cavity as generated by an imaging system such as the for example the imaging system referred to in Figs. 3 and 4 or other imaging systems referred to herein. Thus, the processor circuit 410 may for example communicate with the imaging system 200 (Fig. 3) or the signal processer of a dielectric imaging system (Fig. 4) to obtain the model data. Thus, the model data may actually comprise a ready to use anatomical model (in any data format), but may also include data that need to be and can be processed (for example by the processing circuit) to provide the actual model.

In step 520, performed in parallel to or before or subsequent to step 510, location data is obtained by the processing circuit at the second input. The location data is representative for and dependent on the location of a sensor within the anatomical cavity during the interventional procedure where the sensor is disposed on the balloon therapy catheter such that it can be used to infer the position of the balloon (at least when in inflated condition) within the cavity. The sensor may be any sensor as long as it can be located by the system. It may for example be a magnetic sensor when tracked using a magnetic tracking system. Preferably the sensor comprises one or more of the electrodes of the EP catheter as then it may be tracked by the imaging system as described for example with reference to Figs. 2 and 4. In such case step 520 may optionally comprise, for example, determining a response of the one or more electrodes to an electric field generated by the external electrodes of a dielectric imaging system. This enables a relative position of the one or more electrodes of the EP catheter to be determined with respect to a co-ordinate system defined by the external electrodes of the dielectric imaging system.

The location data comprises or uses balloon positional data relating defined relative positions of the balloon to the location of the sensor of the balloon therapy catheter or to the member tip 130 of the flexible member 134 when it concerns a balloon therapy catheter 140 as described for example with reference to Fig. 1. In the latter case a calibration procedure may be used to determine the location data as will be described herein below. This positional data may be used to relate the relative position of the balloon to the sensor, e.g. to the located or tracked electrodes of the EP catheter.

In other examples, step 520 may comprise obtaining an indication of a location of the balloon therapy catheter from a location system.

The process 500 also comprises a step 530, performed in parallel to or before or subsequent to steps 510 and 520, of obtaining, e.g. at a third input, geometrical data comprising one or more geometrical parameters associated with the inflatable balloon when in inflated state and/or with the balloon in inflated stat. The geometrical parameters may include or represent a set of assumptions of or known geometrical conditions of the balloon from which the size and shape of the balloon may be known or determined. In particular, geometric parameters may comprise any properties or parameter of the balloon that may affect or determine its geometric shape, extent or size of boundary (e.g. in free space), such as a surface area of the balloon, a radius of the balloon, a dimension of the balloon, a volume of fluid in the balloon, a shape of the balloon and so on.

The geometrical parameters may, for example, comprise static parameters of the inflatable balloon (e.g. unchanging properties, such as a length of the inflatable balloon, a surface area of the inflatable balloon, a maximum volume of the inflatable balloon and so on). The geometrical parameters may also/otherwise comprise dynamic parameters (e.g. flow rate of cooling fluid, amount of cooling fluid in the inflatable balloon, predicted volume of the predicted balloon and so on).

Static and/or dynamic properties may, for example, be stored in a separate storage module or memory, such as a remote server. Alternatively, such properties may be stored in a memory of the system or of the processor circuit. The properties may be retrieved upon indication of a user (via the user interface) what type of catheter is attached to the system for use during the procedure. An automatic retrieval of such properties pertaining to a particular catheter, may be done on identification by the system of attachment of the particular kind of catheter to the system. Dynamic properties may, for example, be obtained from a balloon controller (e.g. which controls an operation of the inflatable balloon).

The geometrical properties of the inflatable balloon allow a balloon model with shape and size of the inflatable balloon to be constructed when in inflated state, e.g. to enable a relative distance between different parts of the edge of the inflatable balloon and e.g. a central axis (or center of the balloon on such axis) of the inflatable balloon to be used or calculated. The central axis for example runs along or is parallel and coinciding (at the balloon location) with the axis 620 (Fig. 6) along the length direction of the balloon therapy catheter.

In some examples, e.g. where the process 500 is performed only when the balloon is inflated, the geometrical parameter(s) of the inflatable balloon can be relabeled the geometric parameter(s) of the inflated balloon, for the sake of improved understanding. The receipt of data by the processor has been described in a particular sequence, but this is not necessarily the actual sequence performed. Some of the steps 510 to 530 may be reversed in order or may be partially or entirely parallel. Some of the data processing steps require data from multiple of these steps.

In step 540 a relative position of the balloon (and therewith the balloon therapy catheter) with respect to the anatomical model of the anatomical cavity is determined by the processor circuit and for example at least partly by the data processor from the model data and the location data. Thus, tracking the sensor and transforming the tracked locations of the sensor to locations of the balloon using the balloon position data is performed to relate the balloon location the cavity. Transforming these balloon locations to those with respect to the anatomical model is performed using the anatomical model obtained from the anatomical cavity.

The position of the inflatable balloon may be formed from two components, a determined location and a determined orientation. A location may identify a particular point or points occupied by the inflatable or inflated balloon. An orientation may identify how the inflatable or inflated balloon is angled with respect to these points (and the anatomical cavity or anatomical model thereof). In this way, the position of the inflatable balloon may define how the inflatable balloon is arranged with respect to the anatomical cavity and the anatomical model thereof.

When an EP catheter is used and the location of one or more of its electrodes are tracked by a dielectric system as described herein before, a number of approaches to generate the relative position of the balloon to the anatomical model can be used. For one, the relationship (balloon position data) between the position of the electrode of the EP catheter or a balloon therapy catheter and the balloon may be predetermined and/or calculable. In some embodiments this relation can be obtained from a calibration procedure that establishes the balloon position data. This calibration is preferably performed in situ during the procedure before the actual therapy such as ablation is performed. The retrieved balloon position data are then provided to the processor circuit as part of for example the location data so that it may be used by the processor during performing the method 500.

Thus the tracking may be performed by using a detected electric field to determine a location of the one or more electrodes 424 with respect to the external electrodes of the dielectric imaging system as part of the location data. This information can be used to identify the relative location of the electrode(s) to the anatomical model.

Consider a calibration process for electrode(s) of the EP catheter mounted on an elongate tip that can be retracted into (a sheath of) the balloon therapy catheter as described for example with reference to Fig. 2. The relationship between the electrode(s) and the inflatable balloon can be configured/calibrated by retracting electrodes into the sheath (e.g. shielding the electrodes) so that they are less or unresponsive to electric fields, before extracting the electrodes from the sheath until the most distal electrode becomes responsive to the electric field. The reverse process can additionally or alternatively be performed. At the instance an electrode becomes active (also referred to as valid) it will have just exited the balloon catheter tip (e.g. 130 of Fig 1). The elongate tip can then be locked or fixed in relative position (in registration) with respect to the electrode, by locking or fixing the balloon therapy catheter with respect to the EP catheter using the control handle (this is a know function on such devices), so that a relationship between the position of the electrode(s) and the other components of the balloon therapy catheter is effectively fixed. This enables the relative position between the electrodes and the tip of the balloon therapy catheter to be determined while the electrode can be used for tracking. The location of the balloon and the tip is fixed and known by the design of the catheter and may also be provided as part of the location data. The interrelations between position of the observed electrode with other electrodes on the EP catheter make all electrodes useable for tracking the position of the balloon. For example, the relative location of a center or central axis of the balloon can be calculated from the determined location of the electrode(s).

The location data may also be used to determine the orientation of the balloon therapy catheter with respect to the anatomical model. This can be performed by tracking the movement or path taken by a location sensor mounted on or in registration with the balloon therapy catheter (e.g. electrode(s)) as it moves through the anatomical cavity. This sensor may thus comprise any valid electrodes as determined by the calibration. Thus, by knowing the position of the location sensor and tracking it over time, the orientation of the balloon therapy catheter (and in particular, the inflatable balloon) can be derived. A radial component of a movement or path can be used to account for sideways movement (compared to a back and forth movement).

If a retractable elongate sheath with one or more electrodes mounted thereon is used, it would be beneficial to keep a relationship between the electrodes and the inflatable balloon fixed whilst navigating the balloon into plane. This helps ensure that the path taken by the electrode can be tracked, and therefore the relative orientation of the balloon within the anatomical cavity can be determined.

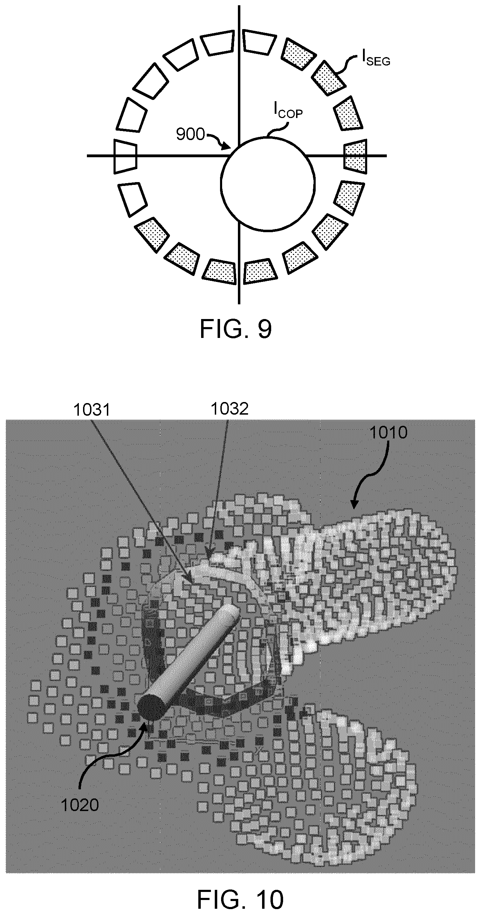

A more detailed description of the calibration procedure and systems and devices needed to perform this calibration procedure referred to herein above is described with reference to Fig. 9 of the non-prepublished provisional European patent application with application number 19206883.1 and filing date of 4 November 2019 (attorney docket number 2019PF00773). A method for visualization of the balloon using such procedure is also described therein and can be used in conjunction with embodiments of the current disclosure. The application 19206883.1 is incorporated by reference in its entirety.

Step 540 may therefore comprise determining orientation information of the balloon from the location data.

However, in other examples, orientation information is obtained/determined in a different manner (e.g. in a separate sub-step). Some examples may derive orientation information using orientation data generated by an orientation sensor, such as an accelerometer or the like, mounted on/in the balloon therapy catheter. An integrated optical shape sensed fiber facilitates tracking of an orientation of a catheter, as does the electromagnetic navigation device described in U.S. Patent Application No. 2016/15764094, which provide suitable alternatives.

Thus, step 530 may comprise further using orientation data when determining the position of the inflatable balloon.

Where a different location system is used, step 530 may comprise correlating a detected position of the balloon therapy catheter to a relative position with respect to the anatomical model. This can be achieved, for example, by converting from a co-ordinate system of the location system to a co-ordinate system of the anatomical model. A relationship between the two co-ordinate systems may be predetermined (e.g. from manufacturers guidance) and/or calculable (e.g. using landmarks or performing a calibration process). Systems for converting between two different co-ordinate systems representing a same anatomical cavity will be apparent to the skilled person.

Step 540 is performed by the processor circuit for example by the data processor.

The process 500 also comprises a step 550 of generating two or more contact indicators. Each contact indicator may represent a prediction of whether a portion of the inflatable balloon is in contact with a boundary of the anatomical cavity.

Step 550 generates each contact indicator by processing the anatomical model, the determined position of the inflatable balloon with respect to the anatomical model (e.g. including balloon location and orientation) and the one or more geometrical parameters associated with the inflatable balloon. It will be appreciated that a determination or prediction of whether or not a particular part or portion of the inflatable balloon is in contact with the boundary of the anatomical cavity can be generated using these input parameters.

The process 500 also comprises a step 560 of outputting a signal carrying contact information responsive to the generated two or more contact indicators. This signal may be provided at an output of the processing circuit.

This signal may, for example, be provided to a display or user interface that generates a display responsive to the generated signal. In some examples, the signal is provided to a storage, for storing generated contact indicators (e.g. for later processing or review). In yet other examples, the signal is provided to an alarm module that, responsive to the signal meeting some predetermined criteria, generates a user-perceptible alarms. Other uses and purposes for the signal will be apparent to the skilled person.

In some embodiments, outputting the signal may include generating a signal carrying contact information carrying the two or more contact indicators and/or additional information derived from the two or more contact indicators.

The signal may, for example, directly control a visual output of a user interface (e.g. carry display data) of the two or more contact indicators and/or additional information derived therefrom, or indirectly control (e.g. by carrying data which is subsequently interpreted by the display or user interface).

In some examples, the signal may comprise any suitable type of communication, such as an electrical signal (e.g., digital electrical signal) representative of image data or other data for influencing the display. For example, the electrical signal may be in a format suitable for display by a display device (e.g., computer monitor, television screen, mobile computing device display, etc.).

Step 550 is further explained. It may comprise, for example, determining for each of a plurality of portions/segments/sections of the inflatable balloon a numerical contact indicator (e.g. a measure) that indicates a relative pressure and/or gap between that portion of the balloon and the bounds of the anatomical cavity.

This may be performed, for example, by calculating or predicting a distance between the edge of the portion of the inflatable balloon and the bounds of the anatomical cavity (as predicted by the anatomical model). A positive distance may indicate a gap between the balloon and the bounds of the cavity. A negative distance may indicate that the balloon is applying pressure to the bounds of the cavity.

The skilled person would appreciate that the distance between the bounds of the anatomical cavity and the (center or central axis of the) balloon therapy catheter (or specifically the inflatable balloon) can be readily predicted using the determined position of the balloon and the anatomical model.

Similarly, the distance between the edge of the (portion of the) inflatable balloon and the (center or central axis of the) balloon therapy catheter (or specifically the inflatable balloon) can be predicted using the one or more geometrical parameters.

In particular, the space occupied by the inflatable balloon can be predicted using the geometrical properties. The position (and optionally orientation) of the inflatable balloon with respect to the anatomical property can be determined from the determined location and the orientation of the balloon. This means that the position of the bounds of the inflatable balloon with respect to the anatomical model can be determined, thereby facilitating determining of the distance between the edge of the balloon and the bounds of the anatomical mode.

These predictions can be used to determine whether or not pressure is applied by the portion of the balloon to the bounds of the anatomical cavity, e.g. and optionally a numerical measure of the same.

More generally, the geometrical parameters and the position of the inflatable balloon can be used to construct a model of the inflatable balloon and derives its position with respect to the anatomical model. This model can be processed to identify whether different portions of the balloon are in contact with the bounds of the anatomical cavity, and therefore generate the two or more contact indicators.

A more complete understanding of step 550 can be obtained with reference to Fig. 6, which illustrates a balloon therapy catheter 420 occluding part of an anatomical cavity 690 (having bounds 695, e.g. tissue walls). The catheter 420 may be one as described with reference to Fig. 1. This it includes a flexible member to which the balloon 422 is attached and within a lumen of the flexible member there is an EP catheter having an electrode 424 indicated in Fig. 6.

The relative position of the electrode 424 to an anatomical model of the anatomical cavity is determined for example using approaches previously described. This effectively enables a model of the inflatable balloon to be constructed and arranged with respect to the anatomical model.

The inflatable balloon is conceptually divisible into two or more (distinct) portions.

In the present example, the balloon is divided into (semi-cylindrical) sections 65(1) - 65(N) along the axis 620 of the inflatable balloon, i.e. each section extending normal to a central axis 620 of the inflatable balloon. Each section is therefore largely parallel to the other sections, and effectively represents a cross-sectional slice of the inflatable balloon with a certain thickness measured along the axis 620. Preferably, the number of sections is no fewer than 10, and more preferably no fewer than 20.

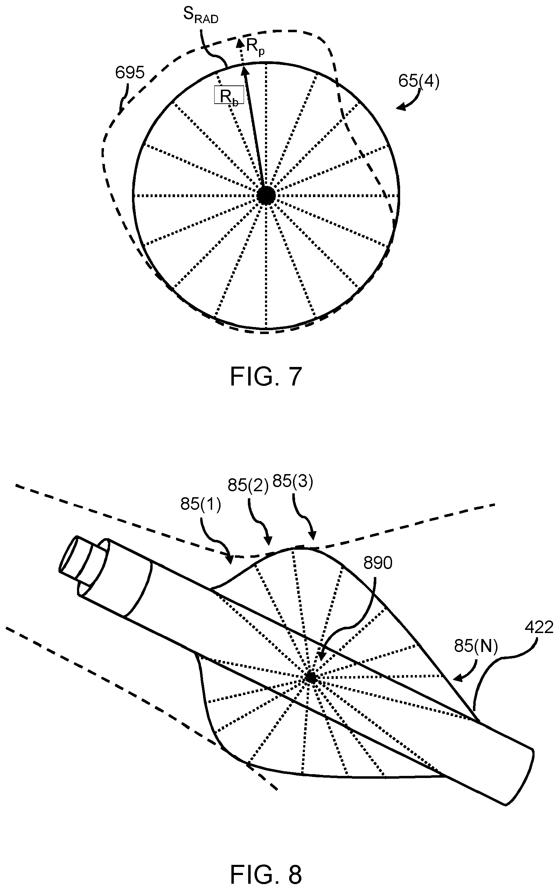

Each section is then sub-divided into a plurality (e.g. 12, 16 or 24) of radial segments, each of which act as a respective portion of the inflatable balloon. Preferably, the number of segments is no fewer than 10, and more preferably no fewer than 20. Each radial segment covers a different range of angles about the central axis, and each radial segment covers a same range of angles about the central axis as a corresponding radial segment of each other section. This segmentation process is best illustrated by Fig. 7, which shows an example of a semi-cylindrical section 65(4) of the inflatable balloon viewed along the axis 620. The section is divided into a plurality of radial segments $S_{RAD}$. Fig. 7 also conceptually illustrates the predicted position of the boundaries 695 of the anatomical cavity (i.e. from the anatomical model) and the inflatable balloon (derivable from the relative position of the balloon therapy catheter and the geometrical property/properties of the inflatable balloon).

Thus, the two or more portions of the inflatable balloon comprise at least two (radial) segments of each of a plurality of cross-sections of the inflatable balloon, where each of the plurality of cross-sections of the inflatable balloon are parallel to one another.

In other words, conceptually, the inflatable balloon can be modelled as a plurality of (almost concentric) cylinders stacked along the length of the central axis 620 of the inflatable balloon, each cylinder having a radius Rb (the radius of the balloon at this section). Each cylinder can be divided into a plurality of segments (e.g. 12, 16 or 24 to provide an even distribution), each of which represents the "portion of the inflatable balloon". Each segment thereby covers a range of angles extending from a particular part of the central axis 620 of the inflatable balloon.

Step 550 may comprise, for each portion, calculating or predicting a distance Rp between the center axis 620 and the bounds of the anatomical cavity ("first distance") for the particular portion of the inflatable balloon. The first distance can be labelled a "mesh distance".

This distance may be taken along a direction of the portion of the inflatable balloon (e.g. a direction originating at the central axis 420 and extending through a center of the portion of the inflatable balloon), as illustrated in Fig. 6.

Alternatively, this first distance may be taken between the central axis and a most proximate part of the anatomical model to the portion of the inflatable balloon (which can be derived based on the known position of the inflatable balloon, the known extent of the inflatable balloon (from the geometrical property/properties) and the anatomical model of the anatomical cavity).

The first distance can be calculated from the anatomical model and the location of the balloon therapy catheter, because the relative position of the central axis 620 with respect to the anatomical cavity (and therefore the bounds of the anatomical cavity) as represented in the anatomical model is known.

Step 550 may also comprise calculating, for each portion, a distance Rb between the center axis 620 and the outer edge of this portion - a "second distance". The direction that this second distance is taken may be the same as the direction in which the first distance is calculated.

This second distance can be calculated using the geometrical properties of the inflated balloon, as these define (or can be used to calculate) the distance between the outer edge of the balloon and the central axis.

A difference between the first distance and the second distance can be used to predict whether or not the portion of the inflatable balloon is in contact with the bounds of the anatomical cavity. Moreover, this difference can be used to predict a gap between the inflatable balloon and the boundaries of the anatomical cavity and/or a pressure applied by the inflatable balloon and the boundaries of the anatomical cavity.

For example, a contact indicator I can be calculated for a portion of the inflatable balloon using the following equation.

$$\mathbf{I} = \mathbf{R_P} - \mathbf{R_b} \qquad\qquad (1)$$

A positive value for I indicates a gap (i.e. there is a leak), and a negative value means occlusion. In particular, a magnitude of a positive value for I represents or is a measure of a gap between the portion of the inflatable balloon to the boundaries of the anatomical cavity. Similarly, a magnitude of a negative value for I represents or is a measure of a pressure applied by the portion of the inflatable balloon to the boundaries of the anatomical cavity.

Thus, the contact indicator I provides an indication of whether the portion of the inflatable balloon is in contact with the bounds of the anatomical cavity. It may also provide an extent of occlusion in terms of pressure applied for one or more portions of the balloon.

Of course, equation (1) may be modified so that a positive value represents occlusion and a negative value represents a gap.

In some examples, generating a contact indicator for a single portion may use information from other (neighboring) portions. For example, a contact indicator for a particular portion may use weighted differences between $R_p$ and $R_b$ of neighboring portions to the single portion.

In some examples, the determined contact indicator I (e.g. from equation (1)) is further processed, e.g. using a step function, to generate a binary contact indicator that predicts whether or not the portion of the inflatable balloon is in contact with the inflatable balloon and/or whether the portion of the inflatable balloon is applying sufficient pressure to avoid leakage.

Thus, a binary contact indicator could be generated for each portion of the inflatable balloon indicating whether or not a measured pressure applied by the portion of the inflatable balloon to the boundaries of the anatomical cavity exceeds some predetermined threshold (e.g. 0 for indicating a contact or some other, non-zero value to indicate a minimum required pressure).

In Figs. 6 and 7, it can be seen that for a particular portion of the inflatable balloon (within section 65(4)), a distance Rp is greater than a distance Rb, indicating that there is a gap between the corresponding portion of the inflatable balloon and the boundary of the anatomical cavity.

Fig. 8 illustrates an alternative approach for conceptually dividing the inflatable balloon 422 into a plurality of portions 81(1) - 85(N).

Rather than each portion representing a radial segment of a semi-cylindrical part of the inflatable balloon, each portion instead represents a volume of the inflatable balloon surrounding a different direction from a predetermined point in the inflatable balloon (e.g. a center 890 of the balloon). Thus, each portion may be analogous to a different spherical segment of the inflatable balloon.

In this scenario, the distance $R_b$ may, for each portion 85(1) - 85(N), be the distance between the predetermined point in the inflatable balloon and the edge of the portion of the inflatable balloon (e.g. along a direction in the middle of the portion of the inflatable balloon). The distance $R_p$ may be distance between the predetermined point in the inflatable balloon and the boundaries of the anatomical cavity.

The foregoing description describes how two or more contact indicators can be generated. Each contact indicator represents a prediction of whether a portion of the inflatable balloon is in contact with a boundary of the anatomical cavity. In some examples, each predictor is a numerical measure indicating a measurement of a predicted pressure and/or gap between the portion of the inflatable balloon and the boundary of the anatomical cavity.

A user interface (e.g. adapted to displaying the anatomical model) may be configured to obtain contact information responsive to the contact indicators, (e.g. from a signal generated in step 560 of process 500) and provide a visual indication of this contact information, e.g. indicating whether or not each portion of the inflatable balloon is in contact with the bounds of the anatomical cavity.

For example, a display may visually represent the anatomical model, e.g. obtained from the dielectric imaging system. The display may further provide a visual indication (e.g. a color, pattern, text or other visual contact indicator) on each area of the anatomical model that corresponds to a respective contact indicator and portion of the inflatable balloon, i.e. an area of the anatomical model that was processed when generating a contact indicator for said portion of the inflatable balloon.

The visual contact indicator may indicate: whether or not the respective portion of the inflatable balloon in in contact with said area of the anatomical model; a size of a leak between the inflatable balloon and said area of the anatomical model (e.g. a value from equation (1); a size of a pressure applied by the inflatable balloon to said area of the anatomical model (e.g. a value from equation (1); and/or whether or not the size of the pressure applied by the inflatable balloon to said area of the anatomical model exceeds a predetermined threshold.

The display may be further configured to provide a visual representation of one or more components of the balloon therapy catheter, such as a visual representation of the inflatable balloon and/or a central axis of the balloon therapy catheter.

Thus, the display may obtain a signal responsive to location information about the relative location of the balloon therapy catheter with respect to the anatomical model, and use this to provide a visual representation of one or more components of the catheter.

It has previously been described how, if a balloon therapy catheter comprises one or more electrodes, the relationship between the position of the electrode(s) and the inflatable balloon can be determined. This same relationship can be used to provide a more accurate visual representation of components of the balloon therapy catheter with respect to the anatomical model (e.g. by appropriately positioning the components with respect to a known or determined position of the electrode(s).

Calibration approaches for determining the relationship between electrode(s) and the components of the inflatable balloon have been described hereinabove.

In some examples, the one or more geometrical properties about the inflatable balloon obtained in step 540 of process 500 can be used to improve the visual representation of an inflatable balloon of the balloon therapy catheter, e.g. to provide a (more accurate) representation of the predicted size of the inflatable balloon.

In other words, the display may provide a visualization of the location and/or shape of the inflatable balloon with respect to the anatomical model of the anatomical cavity. For example, the visualization may be overlaid on an anatomical model generated by the dielectric imaging system.

In some embodiments, the generated two or more contact indicators may be further processed to obtain additional information about the interaction between the inflatable balloon and the boundaries of the anatomical cavity, to enable additional and/or more granular understanding of the operation of the balloon therapy catheter.

The display may be configured to provide a visual representation of this additional information in addition to (or instead of) the visual representation of the two or more contact indicators). The skilled person would be readily capable of controlling or defining the visual representation(s) of any such visual representations, e.g. through appropriate control of the processor circuit (for generating data for display) and the display (for displaying the visual representation).

The further processing of the two or more contact indicators may be performed by the processor circuit of the balloon therapy system or by a processor of the display.

In one example, the two or more contact indicators are processed to identify whether a combination of different portions of the inflatable balloon are in contact, or providing more than a minimum amount of pressure, to the boundaries of the anatomical cavity.

For example, where the inflatable balloon is conceptually divided into a plurality of sections, each of which is formed of a plurality of radial segments (as illustrated in Figs. 6 and 7), the two or more contact indicators may be processed to identify a total pressure applied by a section (e.g. by each cross-section).

In this way, a section contact indicator $I_{SEC}$ may be generated (for each section 65(1) - 65(N)) of the inflatable balloon, illustrated in Fig. 6. The section contact indicator $I_{SEC}$ may be calculated as follows:

$$I_{SEC} = \sum_{i=1}^{M} R_P(i) - R_B(i) \qquad (2)$$

where M represents the total number of segments within a section, i represents a different segment within the section, $R_p(i)$ represents the $R_p$ for that segment and $R_b(i)$ represents the $R_b$ for that segment.

Equation (2) may be modified to include a min-max function, so that each segment can contribute only a predetermined maximum and/or minimum (e.g. no more/less than a value representing 2mm) to the determination of the section contact indicator $I_{SEC}$. Thus, the term $R_P(i) - R_B(i)$ in equation (2) may be replaced by the term $min(U, max(-U, R_P(i) - R_B(i)))$, where U represents a predetermined minimum/maximum value (e.g. a value representing a distance of 2 mm).

This conceptually permits pressure applied by certain segments to account for gaps in other segments (so-called pressure shift), permitting the balloon to expand or contract by this margin.

The section contact indicators may be further processed to identify a "critical section", being a section of the inflatable balloon associated with the largest sum of pressure applied to the boundaries of the anatomical cavity (i.e. the largest negative value of $I_{SEC}$).

Information on the location and identity of the critical section aids a clinician or operator in performing a balloon therapy process, as they are more readily able to identify the location of the balloon therapy catheter that has the largest influence on the balloon therapy of the anatomical cavity.

In the scenario in which the inflatable balloon is conceptually divided into the plurality of largely parallel sections (each of which is formed of a plurality of radial segments (as illustrated in Figs. 6 and 7)), contact indicators may be processed to obtain a segment contact indicator, which indicates a relationship between the combination of corresponding radial segments of two or more different sections.

Radial segments are considered to correspond to one another if they relate to a same range of angles (e.g. between 0° and 15° or between 15° and 30° etc.) about the central axis. Thus, corresponding radial segments are disposed in a stack of radial segments.

A segment contact indicator may, for example, indicate a total sum of pressure applied by the stack of radial segments. Thus, a segment contact indicator $I_{SEG}$ may be generated as follows:

$$\mathbf{I_{SEG}} = \sum_{\mathbf{j=N_1}}^{\mathbf{N_2}} \mathbf{R_P(j)} - \mathbf{R_B(j)} \tag{3}$$

where $N_1$ is a starting section, $N_2$ is the ending section, and j is a segment of the section that corresponds to the stack of radial segments.

Preferably, the critical section (e.g. identified using section indicators generated via equation (2)) is one of the sections falling within the range of $N_1$ to $N_2$.

As one example, a plurality of segment contact indicators can be calculated by using a stack of three or five sections, and calculating the mean pressure and/or leakage for each stack of radial segments within the three/five sections. In preferred examples, the midde section in the stack of three or five is the critical section (as calculated using equation (2)) and the other two/four are its neighbors. For each stack of radial segments, the mean pressure/leakage is calculated (e.g. using equation (3)), to thereby generate a plurality of segment contact indicators. This example helps to smooth out noisy reconstruction data, irregular points distribution, reconstruction resolution that is too coarse for the required number of sectors and so on.

In some examples, the term $R_P(j) - R_B(j)$ in equation (3) is replaced by the term $min(2, max(-2, R_P(j) - R_B(j)))$. This means that each segment in a stack contribute up to -2 or +2 to a total "occlusion sum", which if negative means occlusion and if positive means a gap. The value 2 or -2 may be replaced by other predetermined values, depending upon the user requirements and/or measurement units used (e.g. mm, inches and so on).

As another example, a segment contact indicator may indicate whether the contact indicators of the radial segments in the stack of radial segments meet some predetermined requirement(s).

For example, the predetermined requirement(s) may include a requirement that a gap between each radial segment and the boundaries of the anatomical cavity is less than a predetermined maximum gap (e.g. less than 2mm), and that a total pressure applied by the combination of all radial segments in the stack of radial segments exceeds a predetermined threshold.

As another example, the predetermined requirement(s) may include a requirement that more than a predetermined number (e.g. more than 2 or more than 4) of adjacent radial segments in the same stack provide more than some predetermined amount of pressure to the boundaries of the anatomical cavity.

As yet another example, the predetermined requirement(s) may include a requirement that more than a predetermined number (e.g. more than 2 or more than 4) of adjacent radial segments (preferably including a radial segment of the critical section) in the same stack apply a combined pressure more than some predetermined threshold, and that a gap between each of these adjacent radial segments and the anatomical cavity is less than some predetermined maximum gap (e.g. less than 2mm).

These embodiments enable an indication of whether there is sufficient (according to some predetermined requirements) pressure applied by the inflatable balloon to the boundaries of the anatomical cavity to occlude around the entire circumference of the inflatable balloon (by effectively individually assessing each of a range of angles about the inflatable balloon).

Other example segment contact indicators will be apparent to the skilled person, and may vary depending upon clinical need, implementation details (e.g. the type of balloon therapy being performed) and individual patient requirements.

In another example, the generated two or more contact indicators may be further processed to identify a center of pressure $I_{COP}$ applied by the inflatable balloon to the boundaries of the anatomical cavity.

The center of pressure may, for example, indicate an average position of the pressure applied by the inflatable balloon on the anatomical cavity. This average position may be determined with respect to the central axis of the inflatable balloon, for example, to determine a relative position of the average pressure applied by the inflatable balloon about the central axis.

In particular, the center of pressure may indicate at least an average direction of pressure (e.g. outwardly from the central axis) applied by the inflatable balloon to the boundaries of the anatomical cavity surrounding the inflatable balloon.

The center of pressure may, for example, be visually represented using an arrow or other indicative measure (e.g. the position of a first circle, representing a cross-section of the inflatable balloon within a second, larger circle representing a cross-section of the anatomical cavity).

The skilled person will appreciate various mechanisms for determining the center of pressure. For instance, where the inflatable balloon is conceptually divided into a plurality of parallel (semi-cylindrical) sections, each sub-divided into separate radial segments, then the relative direction of each segment with respect to the central axis is known. Thus, a determined pressure applied by each segment to the boundaries of the anatomical cavity (or a determined gap) can be used to appropriately modify the determined position of the center of pressure.

The center of pressure may also indicate a magnitude of the average direction of pressure. Thus, the magnitude of the average direction of pressure (from the central axis) may be determined.

For example, if it is determined that only a single segment of the inflatable balloon applies pressure to the boundaries of the anatomical cavity, then it can be determined that the center of pressure is in the direction of this single segment.

As another example, if it is determined that all segments of the inflatable balloon apply equal pressure to the boundaries of the anatomical cavity, then it can be determined that the center of pressure is positioned centrally with respect to the central axis.

Fig. 9 is an illustration demonstrating a visual indication of segment contact indicators $I_{SEG}$ and a center of pressure contact indicator $I_{COP}$. Fig. 9 is used to demonstrate the relationship between pressure applied by the portions of the inflatable balloon and the center of pressure.

Crosshairs are also provided to enhance a visual understanding of the inflatable balloon. A central axis of the inflatable balloon is visually represented by the intersection of the crosshairs.

In Fig. 9, each segment contact indicator $I_{SEG}$ represents whether a stack of radial segments meets some predetermined criteria, such as those previously described. For example, a segment contact indicator may indicate whether or not more than a predetermined number of adjacent radial segments in the stack (e.g. including at least a radial segment of a critical section and/or all radial segments) apply more than a predetermined minimum amount of pressure to the boundaries of the anatomical cavity.

The center of pressure contact indicator $I_{COP}$ visually indicates an average direction of (axial) pressure applied by the inflatable balloon to the boundaries of the anatomical cavity. The center of pressure contact indicator $I_{COP}$ also indicates a relative magnitude of the average pressure (in the average direction), with distance from the center 900 of the visual contact indicator increasing as relative magnitude increases.

Previously described embodiments have explained how the processor circuit (and/or a display processor) may generate the two or more contact indicators (for a respective two or more portions of the inflatable balloon) and optionally additional information derived from the two or more contact indicators, such as the section contact indicator(s), the segment contact indicator(s), identification of the critical section and/or the center of pressure contact indicator(s).

Referring back to Figs 3 and 4, it has previously been described how a user interface or display 460 may be configured to receive a signal $S_1$ from the processor circuit 410 and control a visual output of the display based on the received signal.

The signal $S_1$ may, for example, define the visual output of the display (e.g. carry display information), or may comprise data that is further processed by a processor of the display for visual output.

In preferable embodiments, the signal $S_1$ provided by the processor circuit 410 comprises contact information containing the generated two or more contact indicators (which the display is configured to provide a visual representation of) and/or information derived from the generated two or more contact indicators (such as the section contact indicator(s), the segment contact indicator(s), the critical section and/or the center of pressure contact indicator(s)). In other words, the contact information is responsive to the generated two or more contact indicators.

Of course, the signal $S_1$ may instead comprise display data for controlling the display to provide a visual representation of any of these described contact indicators or information. Thus, the $S_1$ would still carry data responsive to the generated two or more contact indicators.

The user interface 460 may be further configured to receive (a signal carrying data of) the anatomical model from the (dielectric) imaging system 200 (which may be transmitted directly to the display or transmitted via the processor circuit 410 as illustrated).

Accordingly, the user interface 460 may also be configured to provide a visual representation of the anatomical model of the anatomical cavity. In preferable examples, the display of the contact indicator(s) and/or additional information derived therefrom may at least partially overlay the visual representation of the anatomical model of the anatomical cavity.

The user interface 460 may be configured to receive (a signal carrying data of) a contact indicator of the position, size and/or shape of the balloon therapy catheter with respect to the anatomical model. The display may be configured to provide a visual representation of the balloon therapy catheter within a display of the anatomical model of the anatomical cavity.

Fig. 10 provides a visual representation of an anatomical model 1010 of an anatomical cavity, a balloon therapy catheter 1020 and a plurality of contact indicators 1031, 1032. The visual representation represents, for example, a visual display provided by a display or user interface.

In particular, the visual representation provides a visualization of an anatomical model 1010 of the LA of a subject, with an indicator of the inflatable balloon being positioned within the PV ostium.

As previously explained, the anatomical model 1010 is generated by the dielectric imaging system, which builds a model or 3D surface of the anatomical model by detecting distortions in generated electrical fields caused by tissue of the bounds of the anatomical cavity.

The position and location of the balloon therapy catheter 1020, with respect to the anatomical model, is derived by tracking the electrode(s) of the balloon therapy catheter using the dielectric imaging system, as previously described. The visualization of the balloon therapy catheter may be based at least partially on the obtained geometric parameters of the inflatable balloon. In an exemplary embodiment, the display provides a live, or real-time view of the balloon therapy catheter, such that a current location of the balloon is shown and updated in real time (in accordance with hardware and processing limitations) as the balloon moves around the anatomical cavity.

In some embodiments, one or more aspects of the visualization 414 of the inflated balloon may be updated or modified by the processor circuit in response to the treatment procedure (e.g., ablation, radiation) commencing. For example, a color, brightness, or other visual aspect may be updated to show that the treatment procedure has begun.

Thus, the display may also receive information about an ongoing treatment (e.g. from the balloon therapy system) and modify the visual representation of the balloon therapy catheter accordingly.

Each visualization of a contact indicator 1031, 1032 provides information about a contact between the inflatable balloon and the boundaries of the anatomical cavity.

For example, a first contact indicator 1031 may indicate that there is a gap between a portion of the inflatable balloon most proximate to the contact indicator and the corresponding (most proximate) boundary of the anatomical cavity. This can be indicated through appropriate color or shading of a portion of the anatomical model.

As another example, a second contact indicator 1032 may identify the "critical section" of the inflatable balloon, previously described. This can be achieved, for example, by overlaying a visual representation of the critical section of the inflatable balloon over the visual representation of the anatomical model 1010.

The second contact indicator 1032 further illustrates, for each segment of the critical section, a relative gap or pressure between that segment and the boundaries of the anatomical cavity. This is illustrated in greyscale (e.g. a lighter color indicating a magnitude of pressure and applied with darker colors indicating a magnitude of a gap between the two).

Other contact indicators are illustrated, but not labelled. For example, the darkest squares indicates locations at which it is predicted that there is a gap between a portion of the inflatable balloon and a most proximate part of the anatomical model. The second darkest squares indicates locations at which it is predicted that there is no gap (or a minimum pressure is applied) between a portion of the inflatable balloon and the most proximate part of the anatomical model. The lightest squares indicate locations about which there is no information about the relationship between the inflatable balloon and the anatomical model.

The present invention facilitates improved understanding of the interaction between the inflatable balloon and the boundaries of the anatomical cavity (e.g. with the tissue wall of the anatomical cavity). This can, for example, credibly assist a clinician in the placement and operation of a balloon therapy catheter within the anatomical cavity of a patient, e.g. to perform PV occlusion.

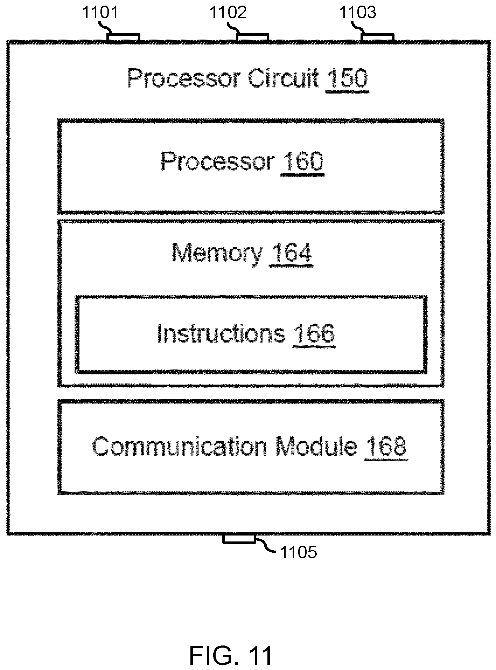

Fig. 11 is a schematic diagram of a processor circuit 150, according to embodiments of the present disclosure.

As shown, the processor circuit 150 may include a (data) processor 160, a memory 164, and a communication module 168. These elements may be in direct or indirect communication with each other, for example via one or more buses.

The processor 160 may include a central processing unit (CPU), a digital signal processor (DSP), an ASIC, a controller, an FPGA, another hardware device, a firmware device, or any combination thereof configured to perform the operations described herein. The processor 160 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 164 may include a cache memory (e.g., a cache memory of the processor 160), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In an embodiment, the memory 164 includes a non-transitory computer-readable medium. The non-transitory computer-readable medium may store instructions. For example, the memory 164, or non-transitory computer-readable medium may have program code recorded thereon, the program code including instructions for causing the processor circuit 150, or one or more components of the processor circuit 150, to perform the operations described herein. For example, the processor circuit 150 can execute operations of the methods 200, 500, 700. Instructions 166 may also be referred to as code or program code. The terms "instructions" and "code" should be interpreted broadly to include any type of computer-readable statement(s). For example, the terms "instructions" and "code" may refer to one or more programs, routines, subroutines, functions, procedures, etc. "Instructions" and "code" may include a single computer-readable statement or many computer-readable statements. The memory 164, with the code recorded thereon, may be referred to as a computer program product.

The communication module 168 can include any electronic circuitry and/or logic circuitry to facilitate direct or indirect communication of data between the processor circuit 150, the catheter 120 and/or the user interface 460. In that regard, the communication module 168 can be an input/output (I/O) device. In some instances, the communication module 168 facilitates direct or indirect communication between various elements of the processor circuit 150 and/or the system (Figs. 3 and 4).

In particular, the communication module 168 may comprise the first input, the second input and the third input, for obtaining the model data, the location data and the geometrical data respectively. The communication module 168 may also comprise an output for providing the signal carrying contact information.

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the Figs.. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

The computer-readable program may execute entirely on a single computer/processor, partly on the computer/processor, as a stand-alone software package, partly on the computer/processor and partly on a remote computer or entirely on the remote computer or server (e.g. using a distributed processor processing system). In the latter scenario, the remote computer may be connected to the computer/processor through any type of network, including a local area network (LAN) or a wide area network (WAN), or the connection may be made to an external computer (for example, through the Internet using an Internet Service Provider).

It will also be understood that the embodiments described above are exemplary and are not intended to limit the scope of the disclosure to a given clinical application. For example the devices, systems, and techniques described above can be used in a variety of balloon ablation applications that involve occlusion of a body cavity or body lumen. For example, in some embodiments, the techniques described above can be used to guide a cryoablation procedure using a cryocatheter comprising a cryoballoon as described above. In other aspects, the techniques described above can be used to guide an RF ablation procedure in which a plurality of RF ablation electrodes positioned on the surface of an inflatable balloon are used to create an electrically-isolating lesion in cardiac tissue. For example, the HELIOSTAR RF balloon therapy catheter, manufactured by Biosense Webster, Inc., includes 10 ablation electrodes positioned on an external surface of the inflatable balloon, and 10 electrodes on a circular mapping catheter positioned distally of the balloon and configured to be positioned inside the PV.

Further, while the ablation procedures are described with respect to the heart and associated anatomy, it will be understood that the same methods and systems can be used to guide ablation procedures in other body volumes, including other regions of interest in the heart, or other body cavities and/or lumens. For example, in some embodiments, the EP guided ablation procedures described herein can be used to guide treatment procedures in any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. The anatomy may be a blood vessel, as an artery or a vein of a patient's vascular system, including cardiac vasculature, peripheral vasculature, neural vasculature, renal vasculature, and/or any other suitable lumen inside the body. In addition to natural structures, the approaches described herein may be used to examine man-made structures such as, but without limitation, heart valves, stents, shunts, filters and other devices in the kidneys, lungs, or any other suitable body volume. Further, the balloon detection and visualization techniques described above can be employed in various applications to determine the location of a balloon. For example, the procedures described above can be used for intravascular balloon-based stenosis treatment, or any other suitable application.

Persons skilled in the art will recognize that the processor circuit, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

**Claims**

1.   A device for assisting a balloon therapy procedure conducted using a balloon of a balloon therapy catheter for contacting a boundary of an anatomical cavity of a subject during the procedure, the device comprising:

- a processor circuit comprising:

- a first input configured to receive model data representative of an anatomical model of the anatomical cavity;
- a second input configured to receive location data representative of a location of the balloon within the anatomical cavity during the procedure;
- a third input configured to obtain geometrical data comprising one or more geometrical parameters associated with the balloon and representative of a balloon model of the balloon in inflated state;

- a data processor communicatively coupled to the first, second and third inputs and configured to:

- determine, using the anatomical model, the balloon model, a relative position of the balloon model with respect to the anatomical model;
- generate, using the anatomical model, the determined relative position and the balloon model, two or more contact indicators each one indicating a state of contact relating to one of two or more surface portions of the balloon when in an inflated state with the boundary of the anatomical cavity; and

- optionally, an output, communicatively coupled to the data processor, the output configured for outputting a signal carrying contact information comprising the two or more contact indicators.

The model data may be generated by an imaging system. The location of the balloon may be a location of the balloon in inflated and/or deflated state. The two or more surface portions at least partially do not mutually overlap. Preferably they do not overlap mutually.

In embodiments or examples, the two or more contact indicators comprises ten or more contact indicators, or even 20 or more contact indicators. A corresponding number of respective balloon portions exists in such cases.

In some embodiments or examples, the state of contact comprises an estimation of whether or not a respective portion of the inflatable balloon is in contact with a boundary of the anatomical cavity.

In embodiments or examples, the balloon is part of and affixed to a balloon therapy catheter which also comprising an EP catheter.

In some embodiments or examples, the processor circuit further comprises an output configured to provide the signal carrying contact information responsive to the two or more contact indicators.

2. The device of claim 1, wherein the two or more contact indicators comprises one or more of: a distance indication of a distance between an edge of the respective portion of the balloon in the inflated state and the boundary of the anatomical cavity and a pressure indication of a pressure applied by the respective portion of the balloon in the inflated state to the boundary of the anatomical cavity.

In some embodiments and examples, each of the two or more contact indicators comprises a distance indication and a pressure indication.

3. The device of any one of the previous claims, wherein the processor circuit is configured to :

   - generate, using the one or more geometrical parameters, the balloon model.

   In embodiments or examples, the generation of the balloon model form the balloon data is performed before the relative position of the balloon model with respect to the anatomical model is determined.

4. The device of any one of the previous claims, wherein the processor circuit is configured to:

   - determine one or more reference points within the balloon envelope of the balloon model;
   - assign or relate to each of the two or more balloon portions a particular surface portion of the balloon model that bounds the balloon portion;
   - for each particular surface portion, define a virtual axis intersecting a determined reference point and a surface point of intersect lying within the particular surface portion and determine a surface portion distance as the distance between the determined reference point and the surface point of intersect;
   - for each particular surface portion, determine a boundary point of intersect of the virtual axis of a particular surface portion with the cavity boundary of the anatomical model and determine a boundary distance as the distance between the determined reference point and the boundary point of intersect defining the virtual axis of that particular surface portion:
   - generate, based on the surface portion distances and the corresponding boundary surface distances, the two or more contact indicators.

5. The processor circuit of claim 4, wherein the step of generating the two or more contact indicators comprises for each one of the indicators calculating a difference between surface portion distance and boundary distance for a particular surface portion.

6. The device of claim 4 or 5, wherein the two surface portions are each part of a balloon segment representing a cross-section of the balloon wherein the cross-sections are parallel to one another, optionally where the reference points, surface intersect point and boundary intersect points are defined such that the virtual axes of different segments are parallel to each other.

In the optional case the reference points are different. The reference points preferably are defined to be part of a central axis parallel to and coinciding with the balloon therapy longitudinal axis within the balloon model.

7. The device of claim 6, wherein each balloon segment comprises at least two radial sections of a cross-section such that a surface portion is defined by a radial section within a segment. Preferably each cross-section extends normal to a central axis parallel to and coinciding with the balloon therapy catheters longitudinal axis within the balloon model.

8. The device of claim 7 wherein the processor circuit is further configured to:

   - be able to communicate with one or more of a plurality of electrodes of an EP catheter and with a plurality of external body patch electrodes for positioning on a subject to apply an electrical field to at least the anatomical cavity using body patch electrical signals;
   - be able to control the external body patch electrodes to apply the electrical field;
   - be able to control the one or more of the plurality of electrodes to:

- generate the location data;
- and, optionally, generate the model data.

**9.** A system for assisting a balloon therapy procedure conducted using a balloon of a balloon therapy catheter for contacting a boundary of an anatomical cavity of a subject during the procedure, the system comprising:

- a device of any of the previous claims wherein the processor circuit comprises an output communicatively coupled to the data processor; and
- a user interface configured to provide an indication of the contact information to the user.

The user interface may be communicatively coupled to the processor circuit at least via the output to this end. The processor circuit can be configured to provide a visual representation of the contact information and the user interface can be configured to provide the visual representation to the user, for example by a display as part of the user interface.

**10.** The system of claim 9, comprising:

- the balloon therapy catheter; and
- an electrophysiology catheter comprising one or more of a plurality of electrodes.

**11.** A method for assisting a balloon therapy procedure conducted using a balloon of a balloon therapy catheter for contacting a boundary of an anatomical cavity of a subject during the procedure, the method comprising:

- receiving, at a first input of a processing circuit, model data representative of an anatomical model of the anatomical cavity ;
- receiving, at a second input of the processing circuit, location data representative of a location of the balloon within the anatomical cavity during the procedure ;
- receiving, at a third input of the processing circuit, geometrical data comprising one or more geometrical parameters associated with the balloon and representative of a balloon model of the balloon in inflated state;
- determining, by a data processor of the processor circuit and communicatively coupled to the first, second and third inputs, using the anatomical model and the balloon model, a relative position of the balloon model with respect to the anatomical model;
- generating, by the data processor, using the anatomical model, the determined relative position and the balloon model, two or more contact indicators each indicating a state of contact of a respective portion of the balloon when in an inflated state with the boundary of the anatomical cavity; and
- optionally, at an output of the processing circuit communicatively coupled to the data processor, a signal carrying contact information comprising the two or more contact indicators.

**12.** The method of claim 11, wherein the two or more contact indicators comprises one or more of: a distance indication of a distance between an edge of the respective portion of the balloon in the inflated state and the boundary of the anatomical cavity and a pressure indication of a pressure applied by the respective portion of the balloon in the inflated state to the boundary of the anatomical cavity.

**13.** The method of any of claims 11 or 12, comprising :

- generating, by the processing circuit, using the one or more geometrical parameters, the balloon model.

**14.** The method of any of the previous claims, wherein generating the two or more contact indicators comprises:

- determining one or more reference points within the balloon envelope of the balloon model;
- assigning or relating to each of the two or more balloon portions a particular surface portion of the balloon model that bounds the balloon portion;
- for each particular surface portion, defining a virtual axis intersecting a determined reference point and a surface point of intersect lying within the particular surface portion and determining a surface portion distance as the distance between the determined reference point and the surface point of intersect;
- for each particular surface portion, determining a boundary point of intersect of the virtual axis of a particular surface portion with the cavity boundary of the anatomical model and determining a boundary distance as the distance between the determined reference point and the boundary point of intersect defining the virtual axis of that particular surface portion:
- generating, based on the surface portion distances and the corresponding boundary surface distances, the two or more contact indicators.

15. The method of claim 14 wherein the step of generating the two or more contact indicators comprises for each one of the indicators calculating a difference between surface portion distance and boundary distance for a particular surface portion.

16. The method of claim 14 or 15 wherein the two surface portions are each part of a balloon segment representing a cross-section of the balloon wherein the cross-sections are parallel to one another, and, optionally, wherein the reference points, surface intersect point and boundary intersect points are defined such that the virtual axes of different segments do not intersect but only cross each other or are parallel.
The reference points preferably are defined to be part of a central axis parallel to and coinciding with the balloon therapy longitudinal axis within the balloon model.

17. The method of claim 16, wherein each balloon segment comprises at least two radial sections of a cross-section such that a surface portion is defined by a radial section within a segment.
Preferably each cross-section extends normal to a central axis parallel to and coinciding with the balloon therapy catheters longitudinal axis within the balloon model.

18. The method of any one of claims 11 to 17, wherein the processing circuit is configured to communicate with one or more of a plurality of electrodes of an EP catheter and with a plurality of external body patch electrodes for positioning on a subject to apply an electrical field to at least the anatomical cavity using body patch electrical signals, the method comprising:

    - controlling the external body patch electrodes to apply the electrical field;
    - controlling the one or more of the plurality of electrodes to:

        - generate the location data;
        - and, optionally, generate the model data.

19. A computer program product comprising code which, when executed by a processor circuit and/or data processor of the device of any of the claims 1 to 9, causes the processor circuit to perform the steps of the method according to any one of steps 13 to 18.

20. A computer-readable medium or data carrier comprising or carrying the computer program product of claim 19.

FIG. 1

FIG. 2

Balloon therapy catheter — 140

200

Imaging system

Signal processor — 450

455

430

MEMORY

432

440

445

411

412

413

PROCESSOR CIRCUIT — 410

DATA PROCESSOR

414

415

S₁

USER INTERFACE — 460

400

FIG. 3

420 — Body electrodes

Balloon therapy catheter — 140

435

455

Signal processor — 450

432

440

445

430

MEMORY

411

412

413

PROCESSOR CIRCUIT — 410

DATA PROCESSOR

414

415

$S_1$

USER INTERFACE — 460

FIG. 4

| | 510 |
|---|---|
| Obtain Anatomical Model | |

↓

| | 520 |
|---|---|
| Obtain Location Information | |

↓

| | 530 |
|---|---|
| Receive Geometrical Parameters | |

↓

| | 540 |
|---|---|
| Determine Relative Position of Balloon Catheter | |

↓

| | 550 |
|---|---|
| Generate Indicators | |

↓

| | 560 |
|---|---|
| Output Signal Responsive to Indicators | |

FIG. 5

FIG. 6

FIG. 7

FIG. 8

FIG. 9

FIG. 10

1101    1102    1103

Processor Circuit 150

Processor 160

Memory 164

Instructions 166

Communication Module 168

1105

FIG. 11

**Europäisches Patentamt**

**European Patent Office**

**Office européen des brevets**

# EUROPEAN SEARCH REPORT

Application Number

EP 20 17 9859

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y<br>A | US 2013/116550 A1 (ISHII HIDEAKI [JP] ET AL) 9 May 2013 (2013-05-09)<br>* paragraphs [0022], [0034] - [0038], [0050] - [0067]; figures 1,2,4,5,6 * | 1-3,9, 19,20<br>10<br>4-8 | INV.<br>A61B5/00<br><br>ADD.<br>A61B18/02 |
| Y | US 2018/280658 A1 (GOVARI ASSAF [IL] ET AL) 4 October 2018 (2018-10-04)<br>* paragraphs [0023] - [0025], [0028] - [0033], [0049] - [0051]; figures 6,7 * | 10 | A61B34/10<br>A61B34/20<br>A61B90/00<br>A61B5/06 |
| A | US 2018/153437 A1 (SCHWARTZ YITZHACK [IL] ET AL) 7 June 2018 (2018-06-07)<br>* paragraphs [0006] - [0008], [0017] - [0019], [0193] - [0196], [0296], [0303] - [0308]; figures 1,2,8C * | 1-20 | |

**TECHNICAL FIELDS SEARCHED (IPC)**

A61B

The present search report has been drawn up for all claims

| Place of search | Date of completion of the search | Examiner |
|---|---|---|
| Munich | 9 November 2020 | Koolen, Ninah |

EPO FORM 1503 03.82 (P04C01)

## ANNEX TO THE EUROPEAN SEARCH REPORT
## ON EUROPEAN PATENT APPLICATION NO.

EP 20 17 9859

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2020

| Patent document cited in search report | | Publication date | Patent family member(s) | | Publication date |
|---|---|---|---|---|---|
| US 2013116550 | A1 | 09-05-2013 | CN | 103118595 A | 22-05-2013 |
| | | | JP | 5981248 B2 | 31-08-2016 |
| | | | JP | 2013031655 A | 14-02-2013 |
| | | | US | 2013116550 A1 | 09-05-2013 |
| | | | WO | 2013005837 A1 | 10-01-2013 |
| US 2018280658 | A1 | 04-10-2018 | AU | 2018202092 A1 | 18-10-2018 |
| | | | CA | 2999639 A1 | 03-10-2018 |
| | | | CN | 108685614 A | 23-10-2018 |
| | | | EP | 3384852 A1 | 10-10-2018 |
| | | | JP | 2018175861 A | 15-11-2018 |
| | | | US | 2018280658 A1 | 04-10-2018 |
| US 2018153437 | A1 | 07-06-2018 | EP | 3294127 A1 | 21-03-2018 |
| | | | US | 2018153437 A1 | 07-06-2018 |
| | | | US | 2019254564 A1 | 22-08-2019 |
| | | | WO | 2016181316 A1 | 17-11-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6002955 A
- EP 1913338 A
- US 201716462388 A
- US 201615764094 A
- US 10278616 B
- US 5983126 A
- WO 2018130974 A
- WO 2019034944 A
- WO 19206883 A

**Non-patent literature cited in the description**

- **BERGER ; MATTHEW et al.** A survey of surface reconstruction from point clouds. *Computer Graphics Forum.,* 2017, vol. 36 (1